# EUROPEAN PATENT SPECIFICATION

(11) **EP 3 898 610 B1**
(45) Date of publication and mention of the grant of the patent: **19.10.2022**
(21) Application number: 18836252.9
(22) Date of filing: 21.12.2018
(51) Int. Cl.: C07D 401/06, A61P 31/04, A61K 31/435

(54) **NOVEL AZA-HEXAPHENE ANTIBIOTIC COMPOUNDS**
NEUARTIGE ANTIBIOTISCHE AZA-HEXAPEN-VERBINDUNGEN
NOUVEAUX COMPOSÉS ANTIBIOTIQUES AZA-HEXAPHÈNE

(43) Date of publication of application: 27.10.2021
(73) Proprietor: Fondazione Istituto Insubrico di Ricerca per la Vita, 21040 Gerenzano (VA) (IT)
(72) Inventor: CARENZI, Giacomo Enrico, 21052 Busto Arsizio (IT); ABBONDI, Monica, 22060 Cabiate (IT); CARRANO, Lucia, 20025 Legnano (IT); BRUNATI, Mara, 22077 Olgiate Comasco (IT); SIGURTA', Alessandro, 21047 Saronno (IT); TARAVELLA, Anna, 21047 Saronno (IT); TURCONI, Paola, 21053 Castellanza (IT)
(74) Representative: Cattaneo, Elisabetta
(86) International application number: PCT/EP2018/086600
(87) International publication number: WO 2020/126035

(56) References cited:
- WO-A1-00/63181
- WO-A1-2006/047891
- WO-A1-2017/093114
- US-A- 5 994 543
- US-A1- 2004 220 195
- ARJUN H BANSKOTA ET AL: "TLN-05220, TLN-05223, new Echinosporamicin-type antibiotics and proposed revision of the structure of bravomicins", THE JOURNAL OF ANTIBIOTICS, vol. 62, no. 10, 14 August 2009 (2009-08-14), pages 565-570, XP055552229, GB ISSN: 0021-8820, DOI: 10.1038/ja.2009.77
- HAIYIN HE ET AL: "Echinosporamicin, a New Antibiotic Produced byMicromonospora echinospora ssp.echinospora, LL-P175", HELVETICA CHIMICA ACTA, vol. 87, no. 6, 1 June 2004 (2004-06-01), pages 1385-1391, XP055552228, CH ISSN: 0018-019X, DOI: 10.1002/hlca.200490126
- ANGIE M. JARRAD ET AL: "Clostridium difficile Drug Pipeline: Challenges in Discovery and Development of New Agents", JOURNAL OF MEDICINAL CHEMISTRY, vol. 58, no. 13, 9 July 2015 (2015-07-09), pages 5164-5185, XP055552230, US ISSN: 0022-2623, DOI: 10.1021/jm5016846

### SEQUENCE LISTING

<110> FONDAZIONE ISTITUTO INSUBRICO DI RICERCA PER LA VITA
<120> NOVEL HEXAPENE ANTIBIOTIC COMPOUNDS
<130> P020861WO-01
<160> 1
<170> BiSSAP 1.3.6
<210> 1
   <211> 1220
   <212> DNA
   <213> Nonomuraea
<220>
   <223> partial sequence of the gene encoding the 16S rRNA of the strain Nonomuraea FIIRV sp. 103/33 DSM 29890
<400> 1
   cggttgggga tgggctcgcg gcctatcagc ttgttggtgg ggtagtggcc taccaaggcg      60
   acgacgggta gccggcctga gagggcgacc ggccacactg ggactgagac acggcccaga     120
   ctcctacggg aggcagcagt ggggaatatt gcgcaatggg cggaagcctg acgcagcgac     180
   gccgcgtggg ggatgacggc cttcgggttg taaacctctt tcagcaggga cgaagttgac     240
   gtgtacctgc agaagaagcg ccggctaact acgtgccagc agccgcggta atacgtaggg     300
   cgcaagcgtt gtccggaatt attgggcgta aagagctcgt aggtggctgg tcgcgtctgc     360
   cgtgaaagcc cgcagcttaa ctgcgggtct gcggtggata cgggccggct agaggtaggt     420
   aggggcaagt ggaattcctg gtgtagcggt gaaatgcgca gatatcagga ggaacaccgg     480
   tggcgaaggc ggcttgctgg gccttacctg acgctgagga gcgaaagcgt ggggagcgaa     540
   caggattaga taccctggta gtccacgctg taaacgttgg gcgctaggtg tggggatctt     600
   ccacgatctc cgtgccggag ctaacgcatt aagcgccccg cctggggagt acggccgcaa     660
   ggctaaaact caaaggaatt gacgggggcc cgcacaagcg gcggagcatg ttgcttaatt     720
   cgacgcaacg cgaagaacct taccaaggct tgacatcgcc cggaaagctg cagagatgtg     780
   gccctcttcg gatcgggtga caggtggtgc atggctgtcg tcagctcgtg tcgtgagatg     840
   ttgggttaag tcccgcaacg agcgcaaccc ttgctccatg ttgccagcac gcctcttcgg     900
   aggtggtggg gactcatggg ggactgccgg ggtcaactcg gaggaaggtg gggatgacgt     960
   caagtcatca tgccccttat gtcttgggct gcaaacatgc tacaatggcc ggtacagagg    1020
   gctgctaaac cgcaaggtgg agcgaatccc taaaagccgg tctcagttcg gattggggtc    1080
   tgcaactcga ccccatgaag tcggagtcgc tagtaatcgc agatcagcaa cgctgcggtg    1140
   aatacgttcc cgggccttgt acacaccgcc cgtcacgtca cgaaagtcgg caacacccga    1200
   agcccgtggc ccaaccacct                                                1220

## Description

### FIELD OF THE INVENTION

The invention concerns novel compounds endowed with potent activity against microbial pathogens, the process for the preparation thereof, and the use thereof in the treatment of bacterial infections. The invention concerns also a new microorganism *Nonomuraea* FIIRV sp. 103/33 deposited on January 19, 2015 in the Deutsche Sammlung von Mikroorganismen und Zellkulturen GmbH, (DSMZ) Inhoffenstraße 7B, 38124 Braunschweig, Deutschland under Budapest Treaty with number DSM 29890.

### STATE OF THE ART

The current emergence of antibiotic resistance leads nowadays to the occurrence of multidrug and pandrug resistant Gram-negative and Gram-positive strains. Severe infections due to methicillin resistant *Staphylococcus aureus* (MRSA) in the United States are decreasing but still numerous, and MRSA remains an important public health problem (Centers for Disease Control and Prevention, 2016). In the last years however, there has been an explosion in the number of MRSA infections reported for populations lacking risk factors for exposure to the health care system (PLoS ONE 8, e52722).

This increase has been associated with the recognition of new MRSA strains, often called community-associated MRSA (CA-MRSA) strains, that have been responsible for a large proportion of the increased disease burden observed in the last decade (Trends Microbiol. 2015, 23: 437-44). CA-MRSA infections tend to occur in previously healthy younger patients. They have been associated predominantly with skin and soft tissue infections but have also been linked to several severe clinical syndromes such as necrotizing pneumonia and severe sepsis. In contrast, health care-associated MRSA (HA-MRSA) strains have been isolated largely from people who are exposed to the health care setting; the patients are older and have one or more comorbid conditions.

The emergence of new CA-MRSA strains has important implications. MRSA infections are expensive and difficult to treat. There are relatively few antibiotic agents available to treat MRSA infections. Moreover, the available agents have important limitations, and the development of new antibiotic classes has slowed. S. aureus isolates that are resistant to each of the few antibacterial drug classes effective against MRSA have been reported, raising the theoretical possibility of untreatable multidrug-resistant (MDR) S. aureus infections (Clinical Microbiology Reviews, 2010, 23 (3): 616-687).

Antibiotic resistance is spreading faster than the introduction of new compounds into clinical practice. To keep our current level of therapeutic efficacy, new antibiotics with new mechanisms of action and structure need to be developed. [Bassetti M., Righi E., "Development of novel antibacterial drugs to combat multiple resistant organisms", Langenbecks Arch Surg. 400(2):153-65 (2015)]. US 2004/220195 discloses antibiotics for use against MRSA.

Natural products have proven to be the most fruitful source of leads for the development of drugs. It has been assessed that ninety five percent of the antibiotics described to date originate from leads discovered by screening natural product extracts or fractions. Many marketed antibacterial drugs are semisynthetic congeners of natural products, and are obtained from the chemical modification of fermentation products (e.g. rifampicin, cephalosporin, oritavancin, dalbavancin). No really new class of broad-spectrum antibiotics has been developed since the quinolones were introduced to the clinic in 1962 and the oxazolidinone linezolid in 2000.

The present invention is directed to overcoming the deficiencies in the prior art. Therefore the object of the present invention is to provide new antibiotics.

### SUMMARY OF THE INVENTION

The present inventors surprisingly found out a compound of Formula (I) 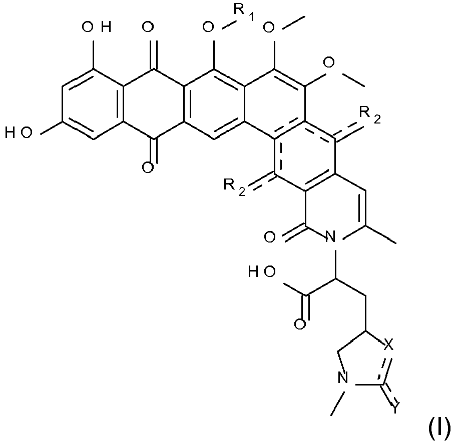 or a pharmaceutically acceptable salt thereof
wherein
R₁ is H or CH₃,
R₂ is O or OH,
the dotted line represents the presence of a single or a double bond, and X is N or NH and Y is NH or NH₂.

The inventors surprisingly found out a compound of Formula (I) or a pharmaceutically acceptable salt thereof, that is a new antibiotic substance of microbial origin, denominated by the inventors as antibiotic FIIRV 103/33, which can be represented by Formula (I).

In a preferred aspect the invention concerns a compound of Formula (I) or a pharmaceutically acceptable salt thereof selected from the group consisting of: 2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionic acid (compound 1 of Formula (I): factor 103/33-F710) 2-(8,10,12-Trihydroxy-6,7-dimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionic acid (compound 2 of Formula (I): factor 103/33-F696) 2-(5,10,12,16-tetrahydroxy-6,7,8-trimethoxy-3-methyl-1,9,14-trioxo-,9,14, -dihydro-1H-2-aza-hexaphen-2-yl)-3-(2-imino-1-methyl-imidazolidin-4-yl)-propionic acid (compound 3 of Formula (I): Factor103/33-F712). 2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-amino-1-methyl-imidazol-4-yl)-propionic acid (compound 4 of Formula (I): factor 103/33-F710a) 2-(8,10,12-Trihydroxy-6,7-dimethoxy-3-methyl-1,5,9,14,16-pentaoxo-5,9,14,16-tetrahydro-1H-2-aza-hexaphen-2-yl)-3-(2-amino-1-methyl-imidazol-4-yl)-propionic acid (compound 5 of Formula (I): factor 103/33-F696a) 2-(5,10,12,16-tetrahydroxy-6,7,8-trimethoxy-3-methyl-1,9,14-trioxo-9,14,-dihydro-1H-2-aza-hexaphen-2-yl)-3-(2-amino-1-methyl-imidazol-4-yl)-propionic acid (compound 6 of Formula (I): factor 103/33-F712a).

The compounds of Formula (I) named as Compounds 1-3 are isomeric forms of compounds 4-6 respectively, since compounds 1-3 present esocyclic C-N bonds instead of endocyclic C-N bonds of compounds 4-6.

The present invention concerns hence compounds of Formula (I) or a pharmaceutically acceptable salt thereof in any enantiomeric and diasteroisomeric forms.

In a further aspect the invention concerns a pharmaceutical composition comprising a compound of Formula (I) and a pharmaceutically acceptable excipient. The composition of the invention can comprise more than one compound of Formula (I) in any amount.

In another aspect the invention concerns a process for the preparation of the compound of Formula (I). The inventors found out a process for producing a compound of Fomula (I) by using a microorganism *Nonomuraea* FIIRV sp. 103/33 DSM 29890 (deposited on January 19, 2015 at the Deutsche Sammlung von Mikroorganismen und Zellkulturen GmbH, (DSMZ) Inhoffenstraße 7B, 38124 Braunschweig, Deutschland under Budapest Treaty under accession number 29890).

Therefore in another aspect the invention concerns the microorganism *Nonomuraea* FIIRV sp. 103/33 deposited as number DSM 29890 on January 19, 2015 in the Deutsche Sammlung von Mikroorganismen und Zellkulturen GmbH, (DSMZ) Inhoffenstraße 7B, 38124 Braunschweig, Deutschland under Budapest Treaty.

In a further aspect, the invention thus concerns an antibiotic compound obtainable by the strain *Nonomuraea* FIIRV sp. 103/33 DSM 29890.

In another aspect the invention hence concerns a use of biologically pure culture of the strain *Nonomuraea* FIIRV sp. 103/33 DSM 29890, for producing an antibiotic compound as a single compound or a mixture thereof.

In a still further aspect the invention concerns a compound of Formula (I) or its pharmaceutically acceptable salt for use as a medicament.

In a further aspect the invention concerns a compound of Formula (I) for use in treating infectious diseases, such as bacterial infectious diseases. In particular according to the present invention, the compounds of Formula (I) are used in the treatment of bacterial infectious diseases caused by Gram-positive bacteria. More preferably the compounds of Formula (I) are used for treating infections from Gram-positive susceptible, resistant and multi-resistant pathogenic bacteria.

### DESCRIPTION OF THE FIGURES

Figure 1A represents the UV spectrum of compound 1 (103/33-F710)
Figure 1B represents the MS (+) spectrum of compound 1 (103/33-F710)
Figure 1C represents the HPLC chromatogram of compound 1 (103/33-F710)
Figure 2 represents the ¹H-NMR spectrum of compound 1 (103/33-F710) in DMSO (500 MHz)
Figure 3 represents the ¹³C-NMR spectrum of compound 1 (103/33-F710) in DMSO (500 MHz)
Figure 4 represents the ¹H-¹³C-HSQC-NMR spectrum of compound 1 (103/33-F710) in DMSO (500 MHz)
Figure 5 represents the ¹H-¹³C-HMBC-NMR spectrum of compound 1 (103/33-F710) in DMSO (500 MHz)

### DETAILED DESCRIPTION OF THE INVENTION

The present invention concerns a compound of Formula (I) 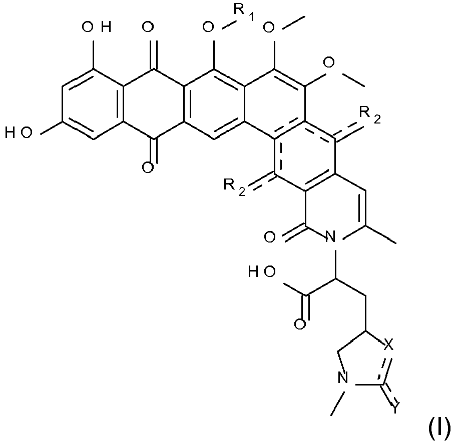 or a pharmaceutically acceptable salt thereof
wherein
R₁ is H or CH₃,
R₂ is O or OH,
the dotted line represents the presence of a single or a double bond, and
X is N or NH and Y is NH or NH₂.

The inventors surprisingly found out a compound of Formula (I) that is a new antibiotic substance of microbial origin, denominated by the inventors as antibiotic FIIRV 103/33, which can be represented by Formula (I).

In a preferred aspect the invention concerns a compound of Formula (I) selected from the group consisting of:
2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionic acid (compound 1 of Formula (I): factor 103/33-F710) 2-(8,10,12-Trihydroxy-6,7dimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionic acid (compound 2 of Formula (I): factor 103/33-F696) 2-(5,10,12,16-tetrahydroxy-6,7,8-trimethoxy-3-methyl-1,9,14,trioxo-,9,14, -dihydro-1H-2-aza-hexaphen-2-yl)-3-(2-imino-1-methyl-imidazolidin-4-yl)-propionic acid (compound 3 of Formula (I): Factor103/33-F712);
2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-amino-1-methyl-imidazol-4-yl)-propionic acid (compound 4 of Formula (I) ): factor 103/33-F710a);
2-(8,10,12-Trihydroxy-6,7-dimethoxy-3-methyl-1,5,9,14,16-pentaoxo-5,9,14,16-tetrahydro-1H-2-aza-hexaphen-2-yl)-3-(2-amino-1-methyl-imidazol-4-yl)-propionic acid (compound 5 of Formula (I) ): factor 103/33-F696a);
2-(5,10,12,16-tetrahydroxy-6,7,8-trimethoxy-3-methyl-1,9,14-trioxo-9,14,-dihydro-1H-2-aza-hexaphen-2-yl)-3-(2-amino-1-methyl-imidazol-4-yl)-propionic acid (compound 6 of Formula (I) ): factor 103/33-F712a).

The present invention concerns hence compounds of Formula (I) in any enantiomeric and diasteroisomeric forms.

In a further aspect the invention concerns a pharmaceutical composition comprising a compound of Formula (I) or a mixture thereof and a pharmaceutically acceptable excipient.

The pharmaceutical composition can be formulated with a pharmaceutically acceptable excipient in an orally administrable form, in a topically administrable form, or in a parenterally administrable form.

The compounds of the present invention, hence, in their pharmaceutically acceptable form, may be administered via oral, topical or parenteral route depending on the treatment to be performed. These compounds can be formulated into different dosage forms according to the route of administration. The preparations for oral administration may be in the form of tablets, capsules, lozenges, liquid solutions or suspensions. As known in the art, tablets, capsules and lozenges may contain usual excipients in addition to the active ingredient, for example extenders such as lactose, calcium phosphate, sorbitol and the like; lubricants such as magnesium stearate, polyethylene glycol (PEG), binding agents such as polyvinyl pyrrolidone, gelatine, sorbitol, acacia, flavoring agents, disintegrating agents and dispersing agents.

Liquid preparations, generally in the form of aqueous or oily solutions or suspensions, may contain conventional additives such as dispersing agents. For topical use, the compounds of Formula (I) of the invention can also be prepared in suitable forms to be absorbed by either the mucous membranes of nose and throat or bronchial tissues, and they may advantageously be in the form of a spray. Topical applications can be formulated as ointments, lotions, gels or powders in hydrophobic or hydrophilic bases.

The pharmaceutical formulation of the invention can be preferably in the form of a capsule, a tablet, a losange, a liquid solution, a liquid suspension, an aqueous solution, an aqueous suspension, an oily solution, an oily suspension, a hydrophobic base ointment, a hydrophobic base cream, a hydrophobic base lotion, a hydrophobic base paint, a hydrophobic base powder, a hydrophilic base ointment, a hydrophilic base cream, a hydrophilic base lotion, a hydrophilic base paint, and a hydrophilic base powder.

In another aspect the invention concerns a process for the preparation of the compound of Formula (I). The inventors found out a process for producing a compound of Formula (I) by using a microorganism *Nonomuraea* FIIRV sp. 103/33 DSM 29890 (deposited on January 19, 2015 at the Deutsche Sammlung von Mikroorganismen und Zellkulturen GmbH, (DSMZ) Inhoffenstraße 7B, 38124 Braunschweig, Deutschland under Budapest Treaty under accession number 29890).

Therefore, in another aspect the invention concerns the microorganism *Nonomuraea* FIIRV sp. 103/33 deposited as number DSM 29890 on January 19, 2015 in the Deutsche Sammlung von Mikroorganismen und Zellkulturen GmbH, (DSMZ) Inhoffenstraße 7B, 38124 Braunschweig, Deutschland under Budapest Treaty.

In a further aspect, the invention thus concerns an antibiotic compound obtainable by the strain *Nonomuraea* FIIRV sp. 103/33 DSM 29890.

In another aspect the invention hence concerns a use of biologically pure culture of the strain *Nonomuraea* FIIRV sp. 103/33 DSM 29890, for producing an antibiotic compound as a single compound or a mixture thereof.

Specifically, the process for the preparation of the compound of Formula (I) comprises the following steps:
(a) cultivating the strain *Nonomuraea* FIIRV sp. 103/33 DSM 29890, under aerobic conditions, in an aqueous nutrient medium comprising a source of carbon, nitrogen and inorganic salts, thus obtaining a culture fermentation broth comprising one or more compounds of Formula (I);
(b) isolating the compound of Formula (I) from the culture fermentation broth comprising one or more compounds of Formula (I);
(c) purifying the isolated compound of Formula (I).

Preferably, the process provides for pre-cultering the strain *Nonomuraea* FIIRV sp. 103/33 DSM 29890 before step a).

In the aqueous nutrient medium of step a) starch, dextrin, glucose, maltose and the like can be the carbon source, soybean meal, peptone, meat extract, casein hydrolysate, tryptone, yeast extract and the like can be the nitrogen source. The aqueous medium can be supplemented with salts capable of providing sodium, potassium, iron, zinc, magnesium, calcium, ammonium, chloride, carbonate, sulphate, phosphate, nitrate and the like ions.

The production strain for the compounds of Formula (I) of step a) is preferably grown in a flask or small fermenter, and the culture is used to inoculate fermentation reactors for the production. The pre-culture medium can be the same or different from that used for the production on an increased scale. According to a preferred aspect, *Nonomuraea* FIIRV sp. 103/33 DSM 29890 is grown on ISP3 agar plates (see Experimental Section) in which the strain forms colonies developing orange vegetative mycelium and globose bodies. The growth temperature for the strain *Nonomuraea* FIIRV sp. 103/33 DSM 29890 is preferably 24-40°C, more preferably 28-32°C. During fermentation, the production of the compounds of Formula (I) preferably occurs within 3-6 days of fermentation.

Step b) provides for the isolation of the compound of Formula (I). Preferably, step b) of isolation of the compound of Formula (I) is carried out by centrifuging or filtering the fermentation broth and recovering the antibiotic from the supernatant or filtered broth according to at least one technique selected from: extraction with a water-immiscible solvent, precipitation by adding a non-solvent or by changing the pH of the solution, absorption chromatography, partition chromatography, reverse phase partition chromatography, ion exchange chromatography, molecular exclusion chromatography, or a combination of two or more of said techniques.

In an advantageous aspect, the step b) of isolation is carried out by separating the mycelium from the supernatant of the fermentation culture broth and extraction of the mycelium with a water-miscible solvent whereby, after the removal of the spent mycelium, a water-miscible solution containing the crude antibiotic compound of Formula (I) is obtained. The compound so obtained is then preferably processed to recover the compound of Formula (I) by means of a technique selected from at least one of: extraction with a solvent, precipitation by adding a non-solvent or by changing the pH of the solution, absorption chromatography, partition chromatography, reverse phase partition chromatography, ion exchange chromatography and molecular exclusion chromatography, or a combination of two or more of said techniques.

In a further aspect the isolation step b) of the process is carried out on the whole strain culture by adding an absorbing resin, maintaining the obtained mixture under stirring until the antibiotic compound of Formula (I) is almost entirely bound to the resin, separating the resin and mycelium by filtration or centrifugation and submitting said separated material to extraction with a water-miscible solvent. From the obtained solution of the antibiotic compound of Formula (I) the product is recovered by at least one of the techniques selected from at least one of: extraction with a solvent, precipitation by adding a non-solvent or by changing the pH of the solution, absorption chromatography, partition chromatography, reverse phase partition chromatography, ion exchange chromatography and molecular exclusion chromatography, or a combination of two or more of said techniques.

In a still further aspect the invention concerns a compound of Formula (I) or its pharmaceutically acceptable salt for use as a medicament.

In a further aspect the invention concerns a compound of Formula (I) for use in treating infectious diseases, such as bacterial infectious diseases. Preferably the bacterial infectious diseases caused by Gram-positive bacteria, more preferably infections caused by Gram-positive susceptible, resistant and multi-resistant pathogenic bacteria.

In a still more preferably embodiment the compound of Formula (I) is used in the treatment of a bacterial infectious disease caused by bacteria of at least one of the following genera: *Enterococci, Staphylococci, Listeriae* and *Bacilli.*

In an advantageous aspect the compound of Formula (I) is used for treating a bacterial infectious disease caused by bacteria selected from the multi resistant strains of *Staphylococcus* spp. and *Enterococcus* spp.

The compound of Formula (I) is used in the treatment of bacterial infectious diseases in a dosage range from 1 to 40 mg of compound of Formula (I) per Kg of body weight.

The section reporting the examples of the present specification provides comparative tests carried out to verify the microbiological selectivity and efficacy of the major compound of Formula (I) compared with known reference compounds. Experimental tests carried out have shown that such compound inhibits the growth of Gram-positive susceptible, resistant and multi-resistant pathogenic bacteria. Representative compound 1 (103/33 F-710) inhibits the growth of Gram-positive susceptible, resistant and multi-resistant pathogenic bacteria, preferably in a concentration from 0.01 to 0.25 µg/ml.

Experimental tests have shown that representative compound 1 (103/33 F-710) does not inhibit replication of HeLa cell at 200 µg/ml. This suggests low to no cytotoxicity.

Other experimental tests have shown that compound 1 (103/33 F-710) can treat an infection by *Staphylococcus aureus* in a murine model with an ED50 of 15 mg/kg.

The isolated compounds of Formula (I) are preferably and advantageously employed as antimicrobial agents against Gram positive bacteria, such as *Staphylococcus* sp, *Enterococcus* sp, *Listeria* sp., *Bacillus* sp. including MDR strains.

As it will be evident form the experimental part the compounds of Formula (I) present in their chemical structure an uncommon fused hexacyclic aromatic scaffold incorporating a 2-pyridone moiety, with a N-linked rare aminoacid belonging to the enduracididine family.

Without being bound to any theory and as it will be evident form the experimental part the inventors deem that the compounds of Formula (I) have substantial peculiarities in the structure of the aromatic scaffold. Specifically they refer to a unique substitution pattern of hydroxyl and methoxy groups and a rarely described non substituted carbon (CH-15 following numbering of compound of Formula (I)) in the "inner side" of the bent scaffold, while all known natural products have an hydroxylated position. The inventors consider the structure of compounds of Formula (I) very surprising in that all other positions in the central fused rings are fully substituted.

The invention will be now detailed by a general method for preparing the strain of *Nonomuraea* and by a more detailed explanation of methods for producing the compounds of Formula (I). Furthermore the invention will be detailed by illustrative and non-limitative examples.

### Experimental part

### Methods of producing the compounds of Formula (I)

### Production strain and general fermentation methods

The production of the compounds of Formula (I) was obtained by cultivating a strain of *Nonomuraea,* such as *Nonomuraea* FIIRV sp. 103/33 DSM 29890. Strain *Nonomuraea* sp. 103/33 was identified on the basis of its 16S rRNA gene sequence and assigned as DSM 29890.

### Partial 16S rRNA Gene Sequence

SEQ ID NO: 1 below shows the partial sequence, consisting of 1220 nucleotides, of the gene encoding the 16S rRNA of the strain *Nonomuraea* FIIRV sp. 103/33 DSM 29890. This sequence was compared with those deposited in public databases, and it is found to be highly related (>99%) to the 16S rRNA sequence of various strains of *Nonomuraea.*

The production of the compounds of Formula (I) was obtained under aerobic conditions in an aqueous production medium containing easily digestible and usable sources of carbon, nitrogen and inorganic salts, such as starch, dextrin, glucose, maltose and the like as the carbon source, soybean meal, peptone, meat extract, casein hydrolysate, tryptone, yeast extract and the like as the nitrogen source. The medium was preferably supplemented with salts capable of providing sodium, potassium, iron, zinc, magnesium, calcium, ammonium, chloride, carbonate, sulphate, phosphate, nitrate and the like ions.

The production strain for the compounds of Formula (I) was grown in a flask or small fermenter, and the culture was used to inoculate fermentation reactors for the production. The pre-culture medium could be the same or different from that used for the production on an increased scale. As a preferred aspect of the pre-culturing step before step a) of the process, *Nonomuraea* FIIRV sp. 103/33 DSM 29890 was grown on ISP3 agar plates in which the strain forms colonies developing orange vegetative mycelium and globose bodies. The growth temperature for the strain *Nonomuraea* FIIRV sp. 103/33 DSM 29890 was preferably 24-40°C, more preferably 28-32°C. During fermentation, the production of the compounds of Formula (I) was monitored by HPLC, and it occurred within 3-6 days of fermentation. The fermentation process was optimized and scaled up several times to 10-liters volume: pH/O₂/temperature conditions, medium parameters and composition were studied to enhance productivity.

Correlation between biomass growth and production of antimicrobial factors was investigated. In a first experiment, one 90 mm strain 103/33 plate was grown for 14 days, then used to inoculate vegetative flasks. One plate was suspended in 20 ml physiological solution (4% inoculum). Flasks were grown for 7 days at 28°C and used to inoculate the 10-liters fermenter (5% inoculum). Low productivity and high biomass were obtained. Production stopped after 75 h. Growth seemed to start again after 70 h. In a second experiment, one 90 mm 103/33 plate was grown for 30 days, then used to inoculate vegetative flasks. Half plate suspended in 20 ml physiological solution (3% inoculum). Flasks were grown for 4 days at 28°C and used to inoculate the 10-liters fermenter (5% inoculum). High productivity and low biomass were obtained. Stationary phase reached after 50 h. Production increased until harvest time. No correlation between growth and production was then observed. The maximum calculated production was 60-70 µg/ml.

Fermentation products were predominantly found in the supernatant. The production as well as the recovery and purification steps can be monitored by a variety of analytical procedures including inhibitory assay against a susceptible microorganism and analytical control using the HPLC or HPLC coupled with mass spectrometry. The fermentation process was easily monitored by HPLC/MS and quantitative methods were setup to measure the actual production. Typical harvest time was 90-96 hours, corresponding to the highest peak area of the produced factors determined by UV. Three different products were detected in the culture fermentation broth, slightly different in the UV/molecular weight/biological activity pattern: the most abundant of them (compound 1: F-710) shows MW = 710, with an estimated yield ranging between 60-70 µg/ml (compound 1 of the invention) . The other two compounds F-696 (compound 2) and F-712 (compound 3) were named according to their molecular weight.

### General Isolation and Purification Protocols for Compounds of the invention

Mycelium/supernatant separation was obtained by centrifugation. Alternatively, filtration of the whole microbial culture can be achieved using suitable paper filters and a Buchner funnel. The resulting pellet containing the mycelium was washed twice with H₂O, which was combined with the supernatant. Typical pH of the pooled solution ranged between 7.0 and 7.5.

The recovery of the compounds from the supernatant of the fermentation broth of the producing microorganism can be conducted by means of a technique selected from at least one of: extraction with water immiscible solvents, precipitation by adding non-solvents or by changing the pH of the solution, absorption chromatography, partition chromatography, reverse phase partition chromatography, ion exchange chromatography, molecular exclusion chromatography and the like or a combination of two or more of said techniques. Specifically, the supernatant coming from the production process of *Nonomuraea* FIIRV sp. 103/33 DSM 29890 was acidified under stirring with an aqueous solution of a mineral acid, 0.5 N to 2 N hydrochloric acid, (a different diluted mineral or organic acid in a pure form, such as formic acid or acetic acid, can be alternatively be used), in the amount required to obtain a solution with a pH ranging between 3.0 and 3.5. When acidification was complete, precipitation of a brown solid occurred, and analysis by HPLC/MS showed that the formed pellet contained the large majority of the 103/33 fermentation products. The resulting supernatant did not contain any of the compounds so it was discarded.

In case the supernatant contains the compounds, the supernatant can be acidified to pH 3 with HCI 1N: precipitation of a pellet occurs and after centrifugation the supernatant can be discarded and the pellet collected.

The pellet was then washed to remove salts/impurities, first with H₂O and then with acetonitrile. This pellet was then extracted several times with acetonitrile added with 5% formic acid, which was successively dried under evaporation. The crude obtained solid contained compound 1 (103/33 F-710) in a 20-25% purity range. In this crude solid, compound 3 (factor 103/33 F-712) can be sometimes detected in small amounts: it can be removed by H₂O₂ oxidation.

Some crude extracts after workup steps in fact showed increased amounts (up to 25-30%) of compound 3 (reduced 103/33 F-712), which was usually found in traces or low amounts (<10-15%) in the fermentation supernatant. A simple reaction with catalytic hydrogen peroxide in H₂O/CH₃CN at 45°C reverted compound 3 (103/33 F-712) back to compound 1 (103/33 F-710). This was monitored by HPLC analysis with UV/PDA detector.

Alternatively, the supernatant can be contacted with an adsorption matrix followed by elution with a polar, water-miscible solvent or a mixture thereof. Examples of adsorption matrixes that can be and were conveniently used in the recovery of the compounds of the invention, are polystyrene or mixed polystyrene-divinylbenzene resins (e.g. M112 or S112, Dow Chemical Co.; Amberlite^{®} XAD2 or XAD4, Rohm & Haas; Diaion HP20, Mitsubishi), acrylic resins (e.g. XAD7 or XAD8, Rohm & Haas), polyamides such as polycaprolactames, nylons and cross-linked polyvinylpyrrolidones (e.g. Polyamide-CC 6, Polyamide-SC 6, Polyamide-CC 6.6, Polyamide-CC 6AC and Polyamide-SC 6AC, Macherey-Nagel & Co., Germany; PA 400, M.Woel AG, Germany), the polyvinylpyrrolidone resin PVP-CL, (Aldrich Chemie GmbH & Co., KG, Germany) and controlled pore cross-linked dextrans (e.g. Sephadex^{®} LH-20, Pharmacia Fine Chemicals, AB). Preferably, polystyrene resins are employed, particularly preferred being the Diaion HP20 resin. In the case of the use of polystyrene resins, polystyrene- divinylbenzene resins, polyamide resins or acrylic resins a preferred eluent was a water-miscible solvent or its aqueous mixtures. In a preferred example, the supernatant was added with Diaion HP-20 resin (1g of hydrated resin per 40 ml of supernatant solution) and kept under stirring for 16 to 48 hours at room temperature. The resin was recovered by sieving (50 mesh). The resin was sequentially washed in a column format with 5-10 column volumes of 30% methanol; no or only minimal product losses occurred in the washes. The product was then eluted in approximately 1-4 column volumes of 100% methanol and successively with a 90/10% methanol/isopropanol mixture. In case some product remained bound to the resin, a final wash with H₂O/CH₃CN/0.1%TFA mixture could collect more. Elution fractions containing the bulk of the products were pooled and evaporated to under vacuum to minimal volume. The resulting solid contained compound 1 (103/33 F-710) in a low (2-5%) purity range so it required further chromatographic purification steps.

Alternatively, the supernatant can be also acidified to pH 3.0 to 3.5 with HCl 1N, and then extracted with a water-immiscible solvent. Examples of water-immiscible organic solvents that can be used in the extraction of the compounds of the invention from the fermentation broth are: alkanols of at least four carbon atoms which may be linear, branched or cyclic such as n-butanol, 1-pentanol, 2-pentanol, 3-pentanol, 1-hexanol, 2-hexanol, 3-hexanol, 1-heptanol, 2-heptanol, 1-octanol, 2-octanol, cyclohexanol, cycloheptanol, cyclooctanol, 1-nonanol, 2-nonanol, 1-decanol, 2-decanol, and 3-decanol and mixtures thereof. Product extraction from the filtered fermentation broth was improved by adjusting the pH at an appropriate value, and/or by adding a proper organic salt forming an ion pair with the antibiotic compound, which was soluble in the extraction solvent. Phase separation can be improved by salting the aqueous phase. When, following an extraction, an organic phase was recovered containing a substantial amount of water, it was convenient to azeotropically distill water from it. Generally, this required adding a solvent capable of forming minimum azeotropic mixtures with water, followed by the addition of a precipitating agent to precipitate the desired product, if necessary. Representative examples of organic solvents capable of forming minimum azeotropic mixtures with water were: n-butanol, benzene, toluene, butyl ether, carbon tetrachloride, chloroform, cyclohexane, and m-xylene; the preferred solvent being n-butanol. Occasionally, the formation of a precipitate at the water/n-BuOH interface was observed, and this had to be kept in the n-BuOH layer. After collecting the organic layer, this was concentrated by evaporation. The resulting solid was resuspended in a 70/30 methanol/H₂O or ethanol/H₂O mixture. The resulting insoluble pellet was collected by filtration, dried and used for the next chromatographic steps.

A small amount (usually less than 20% of total) of compounds can be found in the mycelium. To the fermentation cell solids recovered as described above, a water miscible solvent such as ethanol was added and allowed to contact for 2 to 24 hours (2 ml ethanol/g mycelium). The suspension was centrifuged or filtered and the clear ethanol solution was concentrated to minimal volume under vacuum. The resulting slurry was then suspended in water and extracted with a water immiscible solvent, such as n-BuOH or diluted and contacted with an absorption resin as described above.

Purification of the single products from crude antibiotic FIIRV 103/33 mixture was preferably conducted by means of chromatographic procedures. Examples of these chromatographic procedures are those reported in relation to the recovery step and include also chromatography on stationary phases such as silica gel, alumina, or reverse phase chromatography on silanized silica gel having various functional derivatizations, and eluting with water miscible solvents or aqueous mixture of water-miscible solvents of the kind mentioned above. For instance, preparative HPLC chromatography may be employed, using reverse phase C8 or C18 as stationary phase and a mixture of H₂O/CH₃CN as eluting system. Optional use of buffer solutions or acidic agents such as formic acid or trifluoroacetic acid (TFA) may be required.

In a preferred method, compounds of the invention were separated and purified on a Phenomenex Luna Preparative C18 column 5µ (250 × 21.2 mm) (Phenomenex, Torrance CA) using a gradient or isocratic elution mixture. In a preferred example, starting crude was dissolved in DMSO:H₂O:CH₃CN 1:1:1 (v/v) then injected using a 47 minutes stepwise gradient elution method, including a period of isocratic elution with 38% phase B at 20 ml/min flow rate. Phase B was CH₃CN +0.1% TFA. Phase A was H₂O +0.1% TFA. Pure fractions of compound 1 (factor 103/33 F-710) were pooled and acetonitrile was evaporated, water was extracted with n-BuOH, which was separated and concentrated to minimal volume. Diethyl ether or petroleum ether was then added until precipitation of a red-orange solid occurred. This was filtered, washed and dried under vacuum.

Similarly, compound 2 (103/33 F-696) and compound 3 (103/33 F-712) coming from preparative HPLC were combined, concentrated, then extracted in n-BuOH and added with ethereal solvent so that precipitation occurred and pure compounds were collected by filtration.

### Characterization of the Compounds of the invention

### Physico-Chemical Properties

Solubility of compound 1 (103/33 F-710) was poor in most low to medium- polarity organic solvents, as well as in water from acidic to neutral pH; it was more soluble at basic pH and stable up to pH 8-9, while in alcohols it ranged between 5-10 mg/ml. It was soluble in dimethylsulfoxide (DMSO)(> 50 mg/ml) and dimethylformamide (DMF), and scarcely soluble in methanol (MeOH) and acetone. Compound 2 (F-696) and compound 3 (F-712) showed similar values. Some crystallizations trials were performed on compound 1 (103/33 F-710) in order to obtain suitable crystals for the X-ray structural characterization.

The crystallization experiments were performed in different conditions: pure solvents, stratification of solvents (solvent and anti-solvent), mixture of solvents (solvent and anti-solvent), gel crystallization and presence of co-crystallizing agents like sodium p-toluenesulphonate (STS) and bis(triphenylphosphine) iminium chloride (PPNCI), with the purpose of facilitating the crystals nucleation and growth. The presence of aromatic systems was expected to facilitate the formation of weak interactions with the functional groups of the compound (CH-π, π stacking, etc.). It was not possible to recover single crystals suitable for a single crystal X-ray data collection. In most of the cases, an orange powder was observed, and in various cases spherical aggregates of micrometer size were formed.

### Analytical Data and Structure Elucidation

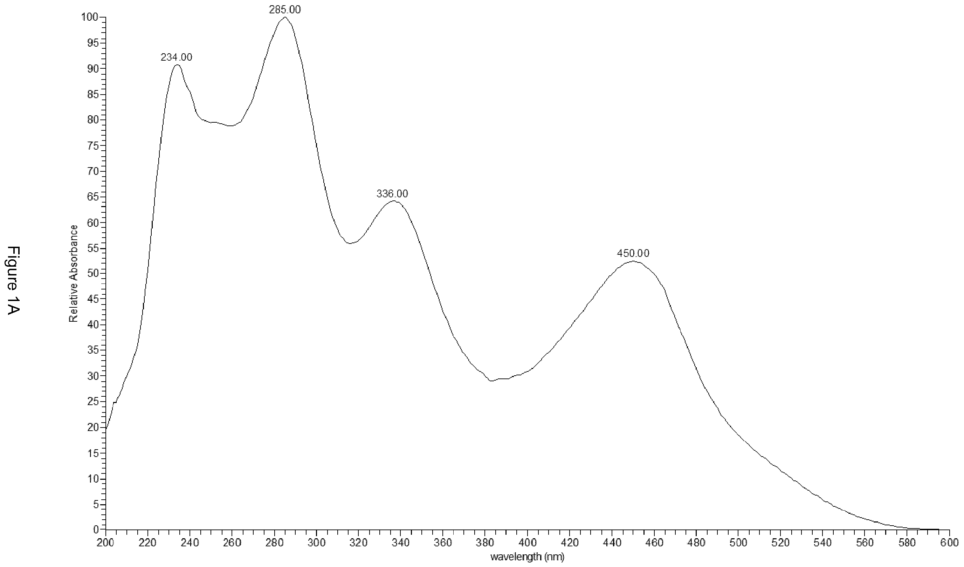

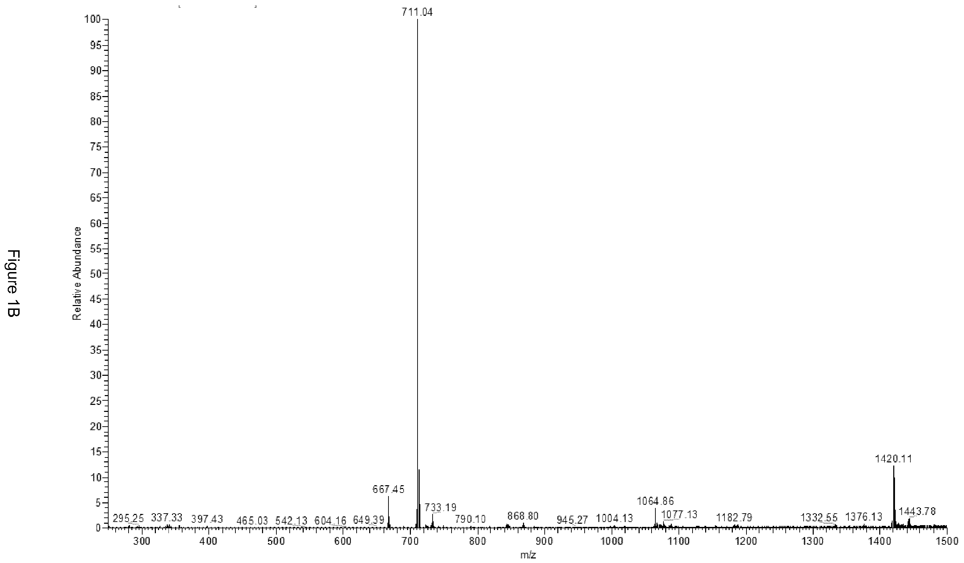

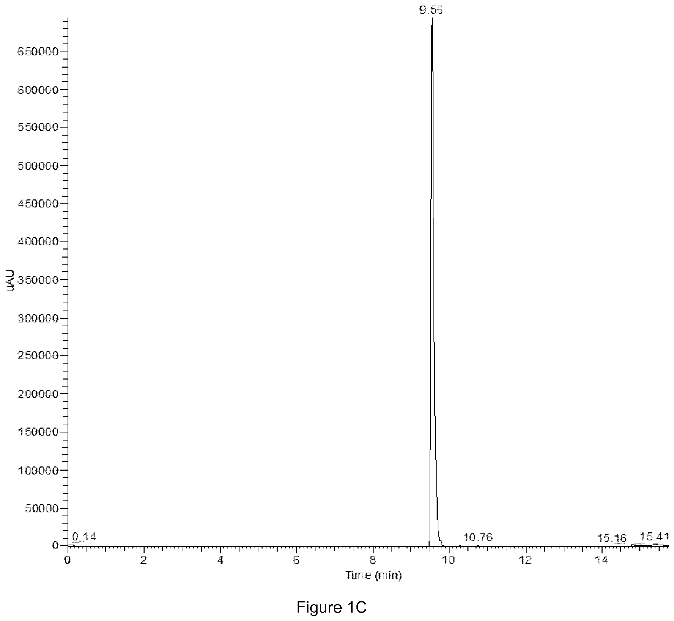

The below reported data show the analytical studies collected on compound 1 (103/33-F710). Accordingly, a purified antibiotic compound 1 (FIIRV 103/33 F-710) obtained as above indicated was obtained as a red-orange powder. A preferred analytical HPLC technique was performed on a HPLC system Accela Instrument (Thermo Fisher Scientific, San Jose, CA) equipped with a chromatographic column Phenomenex Luna C18 5µ (250 × 4.6 mm) eluted at 1 ml/min flow rate and at room temperature. Elution was with a multistep program: Time=0 (30% phase B); Time=2.0 min (30% phase B); Time=11.0 min (70 % of phase B); Time=12.5 min (90% of phase B). Phase A was H₂O + 0.1% HCOOH (v/v) and Phase B was CH₃CN + 0.1% HCOOH (v/v). A Photodiode Array (PDA) detector was used. The effluent from the column was split in a ratio 5:95 and the majority (ca. 950 µl/min) was diverted to PDA detector. Compound 1 (103/33 F-710) in the above reported HPLC conditions had a retention time of 9.40-9.55 minutes. Compound 2 (103/33 F-696) and compound 3 (103/33 F-712) showed a retention time of 9.65-9.75 and 10.70-10.80 minutes, respectively. UV/VIS pattern of compound 1 (103-33-F710) obtained by PDA detection during HPLC analysis showed maximum peaks (282, 336 and 446 nm) typical of a highly conjugated system, including quinone-type aromatics (as reported in Figure 1).

The remaining 50 µl/min of the effluent from the column were diverted to the electrospray ionization (ESI) interface of a LTQ-xl ion trap mass spectrometer (Thermo Fisher Scientific, San Jose, CA). The mass spectrometric analysis was performed under the following conditions: sample inlet conditions: sheath gas (N₂) 45 psi; auxiliary (aux) gas (N₂) 35 psi; capillary heater 275°C; sample inlet voltage settings: polarity both positive and negative; ion spray voltage +/- 3.5 kV; capillary voltage +/- 175V; scan conditions: maximum ion time 200 ms; ion time 5 ms; full micro scan 3; segment: duration 30 min, scan events positive (150-2000 m/z) and negative (150-2000 m/z).

**Table 1: HRMS Fragmentation Results:**

| **Information from LTQ** | | | **FT-HRMS (ESI)** | | **Q-TOF MS** | |
|---|---|---|---|---|---|---|
| **Formula** | **[M+H]+** | **Fragment** | **m/z** | **Error (ppm)** | **m/z** | **Error (ppm)** |
| **C₃₆H₃₁N₄O₁₂** | **711** | | 711.19203 | 1.8 | 711.1956 | 3 |
| C₃₅H₃₁N₄O₁₀ | 667 | -CO₂ | 667.20216 | 2.0 | 667.2048 | 2 |
| C₃₅H₂₈N₄O₁₂ | 696 | -CH₃ | | | | |
| C₃₄H₂₅N₄**O₁₂** | 681 | -C₂H₆ | | | 681.1327 | 20 |
| C₃₃H₂₃N₄O₉ | 619 | -CO₂-H₂O-C₂H₆ | 619.14441 | 2.5 | 619.1471 | 2 |
| C₃₅H₂₉N₄**O₉** | 649 | -CO₂-H₂O | 649.19172 | 1.8 | 649.1951 | 3 |
| C₂₇H₁₆N₄O₉ | 540 | | | | 540.0901 | 2 |

High resolution mass analysis (as in above Table 1) showed the molecular weight was 710. Accurate mass experiments with both ESI and Q-Tof/electron impact gave comparable results, and the suggested molecular formula C₃₆H₃₀N₄O₁₂ was in agreement with the isotopic composition. The high number of unsaturation value (N°u = C - H/2 + N/2 + 1 = 24) was typical for polycyclic aromatic structures.

The molecular ion was also measured on a different HRMS instrument showing 711.19397 m/z (error = 0.172 ppm)

Fragmentation patterns with increasing energy showed few initial neutral losses, including one CO₂ fragment. These data account for a highly conjugated scaffold which fragmentation was poor and required high energy.

Earlier experiments with HRMS showed two smaller neutral fragments with molecular formula C₆H₁₂N₃ and C₄H₈N₃: this helped to assign a crucial part of the polar section.

As an additional note, during crystallization attempts a minor byproduct was isolated from solutions standing in DMSO added with crystallization catalyzers for a long time. Its molecular weight (468) indicated it as a fragment formed by degradation of the entire molecule (710). Accurate mass of fragment 468 and NMR lead to a suggested molecular formula C₂₃H₁₆O₁₁, which was highly in agreement with the formula of the hexacyclic aromatic section. In addition, absence of N atoms in this fragment indicate that the bound polar fragment contains four nitrogens.

1D and 2D NMR experiments include 1H, 13C, 13C-APT, HSQC, 1H-1H-COSY, 1H-1H-TOCSY, 1H-13C-HMBC, 1H-13C-H2BC and 1H-15N-HMBC. Most were performed on a Bruker Avance 500 MHz in DMSO on a 7.2 mg sample which was >99% pure by HPLC/MS. 1,1-ADEQUATE experiments on a 950 MHz with Cryoprobe gave some more updates.

### Structure of compound 1

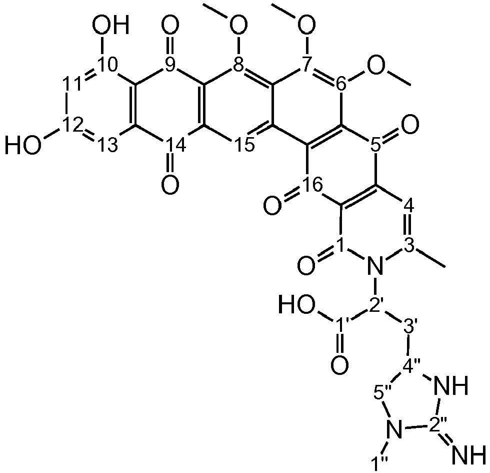

2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionic acid

**Table 2 : assignments of compound 1 (103/33 F-710) according to ¹H, ¹³C, ¹³C-APT and HSQC experiments**

| Atom N | | ¹³C, ppm | ¹H, ppm | | Atom N | | ¹³C, ppm | ¹H, ppm |
|---|---|---|---|---|---|---|---|---|
| | | | | | | | | |
| 1 | C | 158.32 | | | 13a | C | 135.35 | |
| 3 | C | 155.88 | | | 14 | C | 181.90 | |
| 3-Me | CH3 | 22.60 | 2.60 | | 14a | C | 131.52 | |
| 4 | CH | 102.05 | 6.78 | | 15 | CH | 125.90 | 9.58 |
| 4a | C | 144.00 | | | 15a | C | 130.83 | |
| 5 | C | 182.66 | | | 15b | C | 130.90 | |
| 5a | C | 130.20 | | | 16 | C | 182.26 | |
| 6 | C | 151.17 | | | 16a | C | 119.12 | |
| 6-OMe | CH3 | 62.29 | 3.98 | | 1' | C | 170.10 | |
| 7 | C | 155.81 | | | 2' | CH | 56.99 | 5.03 |
| 7-OMe | CH3 | 62.89 | 3.98 | | 3' | CH2 | 35.50 | 2.73 |
| 7a | C | 130.89 | | | 3' | CH2 | 35.50 | 2.05 |
| 8 | C | 159.63 | | | 1"-C | CH3 | 31.76 | 2.89 |
| 8-OMe | CH3 | 63.57 | 3.98 | | 2" | C | 158.54 | |
| 8a | C | 122.55 | | | 4" | CH | 51.24 | 4.10 |
| 9 | C | 186.12 | | | 5" | CH2 | 55.64 | 3.85 |
| 9a | C | 112.06 | | | 5" | CH2 | 55.64 | 3.41 |
| 10 | C | 165.47 | | | O-10 | OH | | 13.28 |
| 11 | CH | 108.77 | 6.65 | | O-1' | OH | | 11.33 |
| 12 | C | 165.24 | | | N-3" | NH | | 8.14 |
| 13 | CH | 107.66 | 7.15 | | N-2" | NH2 | | 7.99 |

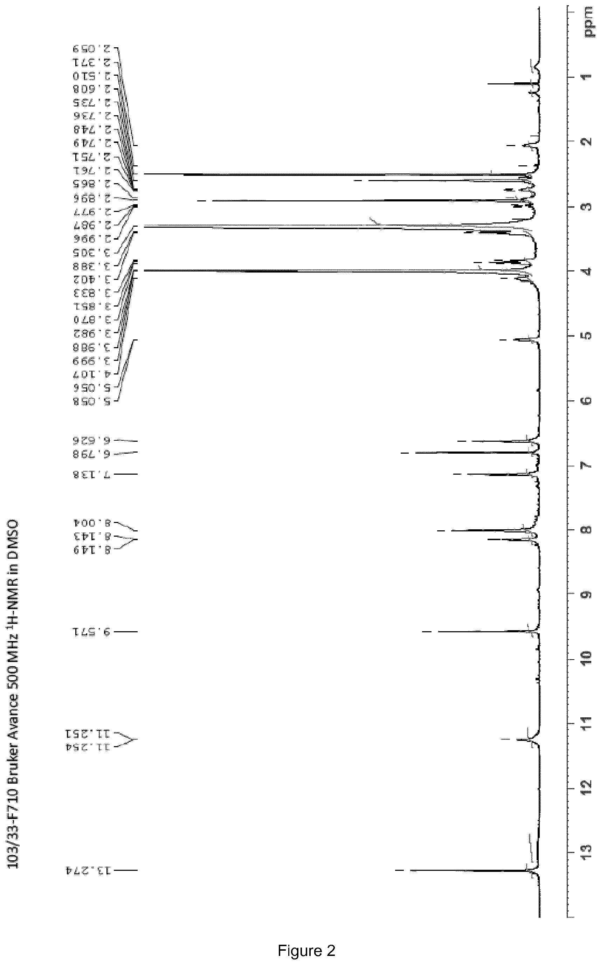

The total number of protons from ¹H-NMR (as represented in Figure 2) was 30, with integration in agreement with the molecular formula C₃₆H₃₀N₄O₁₂ arisen from high resolution mass experiments. The number of exchangeable protons was 5 (corresponding to one OH, one COOH, one NH, and one NH₂). The final product obtained after precipitation was in its trifluoroacetic salt form (NH²⁺) because it was purified by preparative HPLC which contains trace trifluoroacetic acid in the elution phases. Methyl groups were 5 (3 O-CH₃, 1 N-CH₃, 1 aromatic CH₃). The aromatic proton in position 15 at 9.58 ppm coupling with C at 125.90 ppm showed a particular "spatial" situation.

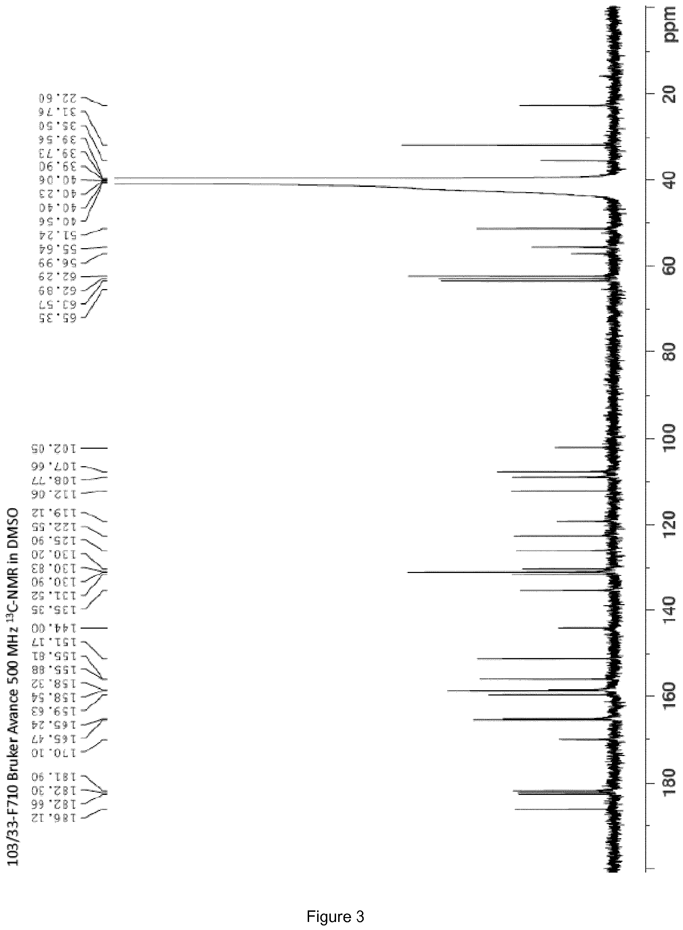

The total number of carbons from ¹³C-NMR (as reported in Figure 3) was 36, in agreement with C₃₆H₃₀N₄O₁₂. One high peak (130.9) integrated for 2C (7a and 15b): an experiment with a Bruker Cryoprobe 950 MHz instrument was able to split these overlapping peaks. Assignment and grouping of C, CH, CH₂, CH₃ was confirmed by APT. Four carbonyl groups were present, and their chemical shift was typical of quinones (180-185 ppm), the first in ring B and the second in ring E. One carboxylic group (C-1', 170 ppm) was in agreement with HRMS fragmentation. Methyl groups were five (3 aromatic O-CH3, 1 N-CH3, 1 CH3). A major key point was the extremely high number of quaternary carbons (23 out of 36), with some overlapping peaks, which made some C-C correlations hard to establish in the aromatic core, as some assignments can be interchanged. In addition, two quinone systems and a series of methoxy and phenolic OH groups complete the polycyclic scaffold, while all the nitrogen atoms were included in a polar side chain.

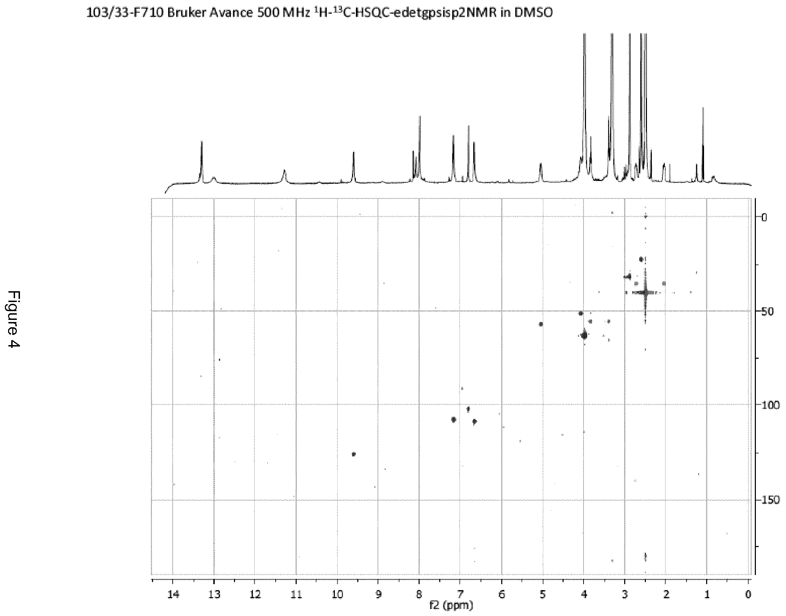

From ¹H-¹³C HSQC correlations (as reported in Figure 4), the aromatic H-15 at 9.58 ppm was very deshielded, while its corresponding carbon showed a very typical aromatic value of 125.90 ppm. Few similar examples were known, including polysubstituted aromatic systems with a particular spatial disposition. Table 2 shows assignments.

¹H-¹H - COSY correlation peaks (Table 3) showed two aromatic protons (H-11 and 13) in meta position, an aromatic methyl associated to one more aromatic CH-4 and the presence of an aliphatic system CH-CH₂-CH-CH₂. 1H-1H - TOCSY also showed correlation for this ring/chain spin system. In addition, methylene protons in position 3' and 5" were diastereotopic, and all chemical shifts were typical for CH- or CH₂-N bonds, which was in agreement with a heterocyclic aliphatic structure comprising nitrogens in this section of the molecule. 1H/15N-HSQC better identifies protonated nitrogens, showing one NH and one NH₂.

**Table 3: Summary of ¹H-¹H correlations**

| Atom N | | H, ppm | Multipl | Integr | C, ppm | H-H COSY |
|---|---|---|---|---|---|---|
| | | | | | | |
| 3-Me | CH3 | 2.60 | s | 3 | 22.60 | H-4 |
| 4 | CH | 6.78 | d | 1 | 102.05 | H-3-Me |
| 11 | CH | 6.65 | s | 1 | 108.77 | H-13 |
| 13 | CH | 7.15 | s | 1 | 107.66 | H-11 |
| 2' | CH | 5.03 | m | 1 | 56.99 | H-3' |
| 3' | CH2 | 2.73 | m | 1 | 35.50 | H-2' |
| 3' | CH2 | 2.05 | m | 1 | 35.50 | H-2', 4" |
| 4" | CH | 4.10 | m | 1 | 51.24 | H-3', 5" |
| 5" | CH2 | 3.85 | m | 1 | 55.64 | H-4" |
| 5" | CH2 | 3.41 | m | 1 | 55.64 | H-4" |

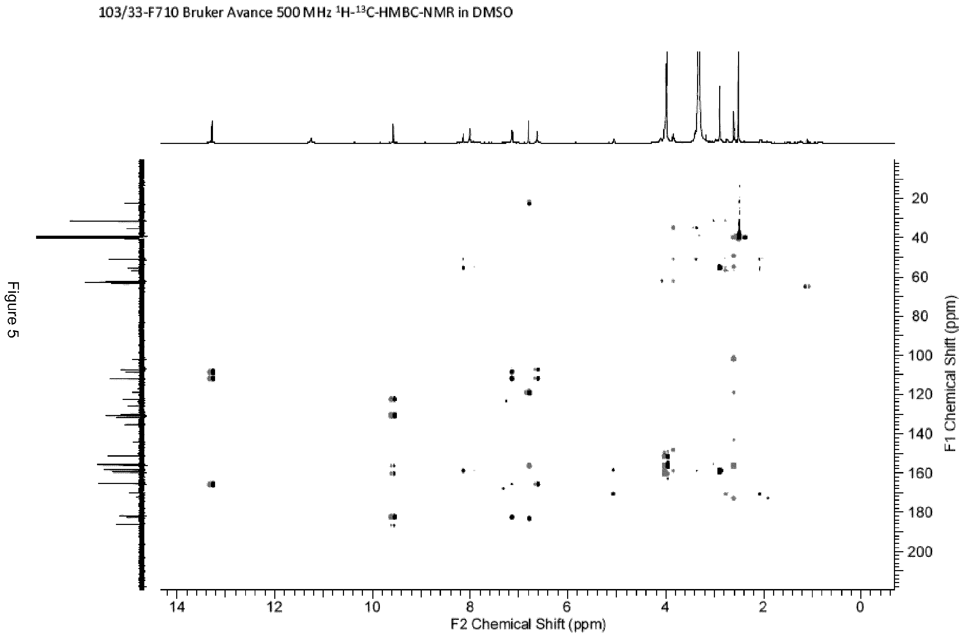

¹H-¹³C HMBC is reported in Figure 5. Together with H2BC, this is crucial to establish C-C correlations despite the low number of protons. The high number of quaternary carbons limited the number of H2BC and HMBC correlations. Some aromatic quaternary carbons were extremely close or overlapping, making assignments problematic: some of them may be interchanged. Some assignments were based on correlations of J-2, J-3 and J-4 cross-peaks by intensity because the spectrum resolution was very high. J-3 cross-peaks were the most intense, while weak cross-peaks were often due to J-2 or J-4 correlations. Six quaternary carbons did not show any HMBC correlation: C-4a, 5a, 13a, 14a, 15a and 16. This was typical in fused rings for bridge-head carbons that were too distant from protons, or close to J-2 protons which distance was poorly sensitive to HMBC. The uncommon H-15 aromatic proton was surrounded by quaternary carbons: a comparison with known polycyclic molecules with an analogous proton showed similarities with some existing scaffolds.

¹H-¹³C-1,1-ADEQUATE confirmed some J-2 C-C correlations involving non-protonated carbons: this helped to distinguish them from other weak HMBC correlations which therefore could be assigned as longer range J-4 cross-peaks. ¹H-¹³C-H2BC highlighted few J-2 cross-peaks between protonated carbons, in agreement with some information coming from COSY and TOCSY data. See Table 4 for all 2D correlations.

¹H-¹⁵N-HETCOR (HMBC) spectrum showed more long-range cross-peaks: typical J couplings were extremely variable in intensity, so they could not reflect real atom proximity. 3 Nitrogen peaks (77, 90, 198 ppm) were visible, all of them correlating with protons in the polar region.

**Table 4: Summary of ¹H-¹³C correlations. Weak correlations are indicated with "(w)".**

| Atom N | | H, ppm | Multipl | Integr | C, ppm | HMBC | H2BC | 1,1-ADEQUATE |
|---|---|---|---|---|---|---|---|---|
| | | | | | | | | |
| 1 | C | | | | 158.32 | | | |
| 3 | C | | | | 155.88 | | | |
| 3-C | CH3 | 2.60 | s | 3 | 22.60 | C-3, 4, 16a(w) | | C-3 |
| 4 | CH | 6.78 | d | 1 | 102.05 | C-3, 4, 5, 16a(w), 2'(w) | | C-3(w), 4a(w) |
| 4a | C | | | | 144.00 | | | |
| 5 | C | | | | 182.66 | | | |
| 5a | C | | | | 130.20 | | | |
| 6 | C | | | | 151.17 | | | |
| 6-OMe | CH3 | 3.98 | s | 3 | 62.29 | C-6 | | |
| 7 | C | | | | 155.81 | | | |
| 7-OMe | CH3 | 3.98 | s | 3 | 62.89 | C-7 | | |
| 7a | C | | | | 130.89 | | | |
| 8 | C | | | | 159.63 | | | |
| 8-OMe | CH3 | 3.98 | s | 3 | 63.57 | C-8 | | |
| 8a | C | | | | 122.55 | | | |
| 9 | C | | | | 186.12 | | | |
| 9a | C | | | | 112.06 | | | |
| 10 | C | | | | 165.47 | | | |
| 11 | CH | 6.65 | s | 1 | 108.77 | C-9a, 10, 13 | | C-10 |
| 12 | C | | | | 165.24 | | | |
| 13 | CH | 7.15 | s | 1 | 107.66 | C-9a, 10 (w), 11, 14 | | C-12, 13a |
| 13a | C | | | | 135.35 | | | |
| 14 | C | | | | 181.90 | | | |
| 14a | C | | | | 131.52 | | | |
| 15 | CH | 9.58 | s | 1 | 125.90 | C-7(w), 7a, 8(w), 8a, 9(w), 14, 15b | | C-14a(w) |
| 15a | C | | | | 130.83 | | | |
| 15b | C | | | | 130.90 | | | |
| 16 | C | | | | 182.26 | | | |
| 16a | C | | | | 119.12 | | | |
| 1' | C | | | | 170.10 | | | |
| 2' | CH | 5.03 | m | 1 | 56.99 | C-1, 3(w), 1', 3'(w), 4"(w) | C-3' | |
| 3' | CH2 | 2.73 | m | 1 | 35.50 | C-1'(w) | C-2' | C-4" |
| 3' | CH2 | 2.05 | m | 1 | 35.50 | C-1'(w) | C-4" | |
| 1" | CH3 | 2.89 | s | 3 | 31.76 | C-2", 5" | | |
| 2" | C | | | | 158.54 | | | |
| 4" | CH | 4.10 | m | 1 | 51.24 | C-2'(w), 2"(w) | C-3', 5" | C-3' |
| 5" | CH2 | 3.85 | m | 1 | 55.64 | C-3', 2"(w) | C-4" | C-4" |
| 5" | CH2 | 3.41 | m | 1 | 55.64 | C-3', 2"(w) | C-4" | C-4" |
| O-10 | OH | 13.28 | s | 1 | | C-9a, 10, 11 | | |
| O-1' | OH | 11.33 | bs | 1 | | | | |
| N-3" | NH | 8.14 | s | 1 | | C-2", 4"(w), 5" | | |
| N-2" | NH2 | 7.99 | s | 2 | | | | |

As a result, the molecule was composed of a bent "hexaphene" aromatic section and a bound polar aminoacid.

Similarly, the other two compounds (compound 2 (103/33-F696) and compound 3 (103/33-F712)) detected in the fermentation process were isolated and characterized by MS/MS and NMR analysis. Experimental details can be found in the examples section.

### EXAMPLES

The Examples set forth below are for illustrative purposes only and are not intended to limit, in any way, the scope of the present invention.

### Example 1. Analytical methods and materials

Unless otherwise noted, reagents and solvents were used as received from commercial suppliers. Nuclear magnetic resonance (NMR) spectra were obtained on Bruker Avance spectrometer at 500MHz. Spectra were given in ppm (δ) and coupling constants, J, are reported in Hertz. Tetramethylsilane (TMS) is used as an internal standard.

Mass spectra were collected using as LC-MS instrument a Thermo Scientific LTQ-xl instrument (Thermo Fisher, Scientific Inc CA,USA) fitted with a ion trap electrospray ionization (ESI) source.

Accurate mass measurement were collected using a ESI-FT-ICR Solarix Instrument (Bruker Daltonics, Germany, GmbH). HPLC analyses were obtained using a Luna C18(2) column (250x4.6 mm, Phenomenex, Torrance, Calif.) or a Symmetry C18 column 250×4.6 mm, (Waters; Milford MA, USA) with Thermo Scientific Accela PDA detector or UV detection at 223 nm using a standard solvent gradient program where phase A (A) was water with 0,1% formic acid, and phase B (B) was CH₃CN with 0,1% formic acid. Here below is reported the solvent gradient program of method 1. Method 1 (analytic):

| Time (min) | Flow (ml/min) | %A | %B |
|---|---|---|---|
| 0 | 1.0 | 70 | 30 |
| 2 | 1.0 | 70 | 30 |
| 11 | 1.0 | 30 | 70 |
| 12.50 | 1.0 | 10 | 90 |
| 13.75 | 1.0 | 10 | 90 |
| 14.50 | 1.0 | 70 | 30 |
| 17 | 1.0 | 70 | 30 |

The preparative HPLC was carried out by using a Shimadzu AP-20 liquid chromatograph (Shimadzu Corporation, Japan). UV data were acquired at 280 nm and 330 nm and the below reported solvent gradient program was used: (A) was water with 0,1% trifluoroacetic acid, and B was CH₃CN with 0.1 % trifluoroacetic acid.

**Method 2 (preparative):**

| Time (min) | Flow (ml/min) | %A | %B |
|---|---|---|---|
| 0 | 8.0 | 62 | 38 |
| 4 | 8.0 | 62 | 38 |
| 6 | 20.0 | 62 | 38 |
| 20 | 20.0 | 62 | 38 |
| 22 | 20.0 | 53 | 47 |
| 28 | 20.0 | 53 | 47 |
| 35 | 20.0 | 10 | 90 |
| 38 | 20.0 | 10 | 90 |
| 41 | 20.0 | 80 | 20 |
| 45 | 20.0 | 80 | 20 |
| 48 | 20.0 | 62 | 38 |
| 51 | 20.0 | 62 | 38 |

**Method 3 (preparative):**

| Time (min) | Flow (ml/min) | %A | %B |
|---|---|---|---|
| 0 | 8.0 | 62 | 38 |
| 4 | 8.0 | 62 | 38 |
| 6 | 20.0 | 62 | 38 |
| 22 | 20.0 | 62 | 38 |
| 35 | 20.0 | 10 | 90 |
| 38 | 20.0 | 10 | 90 |
| 41 | 20.0 | 80 | 20 |
| 45 | 20.0 | 80 | 20 |
| 48 | 20.0 | 62 | 38 |
| 51 | 20.0 | 62 | 38 |

### Example 2.

Fermentation method for *Nonomuraea* FIIRV sp. 103/33 DSM 29890 Nonomurea FIIRV 103/33 DSM 29890 was maintained on oatmeal agar (ISP Medium3) plates for 2-4 weeks at 28°C. The microbial content of one plate was homogenized with 20 ml of physiological solution and 3 ml of suspension were used to inoculate 500ml Erlenmeyer flasks containing 100ml of seed medium (AF/MS) which was composed of (g/l): 20 g dextrose monohydrate, 2 gr yeast extract, 8 gr soybean meal, 1 gr of NaCl and 4 gr CaCO₃. The medium was prepared in deionized-water and pH adjusted to 7.3 prior to sterilization at 121°C for 20 minutes. The inoculated flasks were grown at 28°C on a rotary shaker operating at 200 rpm. After 96 hours, 5% of this culture was inoculated into New Brunswick BioFlo ^{®} 310 fermenter with 10 l of fermentative medium (INA5) which was composed of (g/L) : 15 g soybean meal, 2 g NaCl, 5 g CaCO₃ and 30 ml glycerol. All components were dissolved in deionized-water at pH7 and sterilized at 121°C for 20 minutes. Fermentation parameters were: temperature 28°C, agitation 400 rpm and air at 0.3 vvm.

The production of the antibiotic FIIRV 103/33 was monitored by HPLC analysis of samples coming from the supernatant, following HPLC Method 1 described in Example 1. The fermentation process was stopped when the major reached the maximum.

### Example 3:

Purification protocol for compound 1 (103/33-F710) by acidification of the supernatant and pellet extraction

Mycelium/supernatant separation was obtained by centrifugation. The supernatant (10.5 L) was acidified under stirring with an aqueous solution of 1N hydrochloric acid (140 ml) to pH 3.0. Precipitation of a pellet occurred; after centrifugation the supernatant was discarded and the pellet was collected. This was then washed to remove salts/impurities, first with H₂O and then with acetonitrile. This pellet was then extracted several times with acetonitrile added with 5% formic acid (5 × 1.5 L), which was successively dried under evaporation. The crude obtained solid (1.6 g) contained compound 1 (103/33 F-710) in a 20-25% purity range. The solid was then resuspended in H₂O/CH₃CN/0.1% TFA and reacted with a catalytic amount of 30% hydrogen peroxide (2 ml) at 45°C in order to revert trace reduced compound 3 (103/33 F-712) back to compound 1 (103/33 F-710). The reaction was complete after 4h. The solution was filtered and evaporated to dryness.

Compound 1 (103/33-F710) was then separated from other compounds by purification on a Phenomenex Luna Preparative C18 column 5µ (250 × 21.2 mm) (Phenomenex, Torrance CA). Starting crude obtained above (1.6 g) was dissolved in DMSO:H₂O:CH₃CN 1:1:1 (v/v, 60 ml) then 4 ml were injected using a 51 minutes stepwise gradient elution method, including a period of isocratic elution with 38% phase B from 0 to 22 min at 20 ml/min flow rate, and a gradient elution from 38 to 90% phase B from 22 to 35 min. Then the column was equilibrated back to 38% phase B. Phase B was CH3CN +0.1% TFA. Phase A was H₂O +0.1% TFA. This process was repeated for 15 times. Pure fractions of compound 1 (103/33 F-710) were pooled and acetonitrile was evaporated, water was extracted with n-BuOH, which was separated and concentrated to minimal volume. Diethyl ether was then added until precipitation of a red-orange solid occurs. This was filtered, washed and dried under vacuum. Yield: 270 mg.

Compound 1 (103/33-F710): red/orange powder, m.p. >265°C; UV (MeOH) λ max 282, 336 and 446 nm; MS (ESI) m/z 711.19 (M + H+); 1H-NMR (500 MHz, DMSO): δ (ppm) 13.28 (bs, 1H), 11.33 (bs, 1H), 9.58 (s, 1H), 8.14 (s, 1H), 7.99 (s, 2H), 7.15 (s, 1H), 6.78 (s, 1H), 6.65 (s, 1H), 5.03 (m, 1H), 4.10 (m, 1H, 3.98 (s, 9H), 3.85 (m, 1H), 3.41 (m, 1H), 2.89 (s, 3H), 2.73 (m, 1H), 2.60 (s, 3H), 2.05 (m, 1H); 13C-NMR (500 MHz, DMSO): δ (ppm) 186.12, 182.66, 182.26, 181.9, 170.10, 165.47, 165.24, 159.63, 158.54, 158.32, 155.88, 155.81, 151.17, 144.00, 135.35, 131.52, 130.90, 130.89, 130.83, 130.20, 125.9, 122.55, 119.12, 112.06, 108.77, 107.66, 102.05, 63.57, 62.89, 62.29, 56.99, 55.64, 51.24, 35.5, 31.76, 22.60. See tables for assignments.

### Example 4:

Purification protocol for compound 1 (103/33-F710) by acidification of the supernatant and organic solvent extraction

The supernatant (2.16 L) was acidified to pH 3.0 with hydrochloric acid as described above, and then extracted with water-immiscible solvent n-butanol (2 × 1.1 L). Occasionally, the formation of a precipitate at the water/n-BuOH interface was observed, and this was kept in the n-BuOH layer. After collecting the organic layer, this was concentrated by evaporation. The resulting solid was resuspended in a 70/30 methanol/H₂O or mixture. The resulting insoluble pellet (1.62 g) was collected by filtration, dried and used for the next chromatographic steps.

### Example 5:

Purification protocol for compound 1 (103/33-F710) by contact of the supernatant with absorption resin

The supernatant (9.0 L) was added with Diaion HP-20 resin (1g of hydrated resin per 20 ml of supernatant solution) and kept under stirring for 40 hours at room temperature. The resin was recovered by sieving (50 mesh). The resin was sequentially washed in a column format with 5-10 column volumes of 30% methanol; no or only minimal product losses occured in the washes. The product was then eluted in approximately 4 column volumes of 100% methanol and successively with 4 column volumes of 90/10% methanol/isopropanol mixture. Should some product remain bound to the resin, a final wash with H₂O/CH₃CN/0.1%TFA mixture can collect more. Elution fractions containing the bulk of the product were pooled and evaporated under vacuum to dryness. The resulting solid (3.56 g) contained compound 1 (103/33 F-710) in a low (2-5%) purity range. This was submitted to further chromatographic purification steps (preparative HPLC) as described above.

### Example 6:

Purification protocol for compound 1 (103/33-F710) by mycelium extraction with a water miscible solvent

A small amount (usually less than 20% of total) of compounds according to Formula (I) may be found in the mycelium. To the fermentation cell solids recovered as described above (121.6 g of mycelium from 1.68 L supernatant), a water miscible solvent such as ethanol was added and allowed to contact 4 hours (2 ml ethanol/g mycelium, 245 ml). The suspension was centrifuged or filtered and the clear ethanol solution was concentrated to dryness under vacuum. The resulting solid (1.3 g) was then suspended in water and contacted with an absorption resin as described above.

### Example 7:

Biological activity of compound 1 (103/33-F710)

Antibacterial activity. The antimicrobial activity of the antibiotic 103/33-F710 was determined by the broth microdilution method according to the procedures published by the Clinical and Laboratory Standards Institute (CLSI), formerly the National Committee for Clinical Laboratory Standards (NCCLS) (document M07-A9). Microorganisms were grown in either cation-adjusted Mueller Hinton Broth (*Staphylococci, Enterococci, Moraxella catarrhalis, Escherichia coli*), Todd Hewitt medium (*Streptococci*) or RPMI 1640 medium for *Candida albicans* on 96-well microtiter plates. Each bacterial strain was inoculated with 2-5×10⁵ CFU/ml, *Candida albicans* with 10⁴ CFU/ml. Cultures were incubated at 35°C in air. After 18-24 hours visual readings were performed and MICs (Minimal Inhibitory Concentrations) determined. The effect of 30% bovine serum was determined under the same experimental conditions. The strains used were clinical isolates or strains from American Type Culture Collection (ATCC). The antibiotic 103/33-F710 was dissolved in DMSO to obtain a 1000 µg/ml stock solution, and subsequently diluted in water to obtain working solution. The results are shown in table 5.

**Table 5: Antimicrobial activity reported as MIC (µg/ml) of ID103/33-F710**

| MIC (µg/ml) | | | |
|---|---|---|---|
| | **103/33 - 710** | **Vancomycin** | **Ciprofloxacin** |
| L1400 *S*. *aureus* HA-MRSA | 0.03 | 1 | 0.125 |
| VA80201 *S*. *aureus* CA-MRSA | 0.015 | 1 | 8 |
| L4061 *S*. *aureus* MRSA - GISA | 0.06 | 4 | 64 |
| L100 *S*. *aureus* ATCC6538P | 0.03 | 1 | 0.25 |
| L819 *S*. *aureus* Smith | 0.06 | 1 | 0.06 |
| L1577 *S. epidermidis* | 0.06 | 1 | 0.125 |
| L1730 *S. haemolyticus* | 0.03 | 1 | 0.125 |
| L1729 *S. haemolyticus* MDR | 0.016 | 2 | 8 |
| L49 *S*. *pyogenes* C203 | 16 | 0.5 | 0.25 |
| L44 *S*. *pneumoniae* | 16 | 0.5 | 0.5 |
| VA8101 *S*. *agalactiae* (Group B) | 8 | 0.5 | 0.5 |
| L568 *E. faecium* | 2 | 2 | 2 |
| L569 *E. faecium* VanA | 2 | > 128 | 2 |
| L4187 *E. faecium* VanA - Lnz R - Cpx R | 2 | > 128 | 64 |
| L559 *E. faecalis* | 0.03 | 0.5 | 0.5 |
| L560 *E. faecalis* VanA | 0.03 | > 128 | 0.5 |
| VA801801 *L. monocytogenes* | 0.06 | - | - |
| VA827901 *B. cereus* | 0.06 | - | - |
| VA645601 *B. clausii* | 0.125 | - | - |
| L47 *E. coli* | > 256 | > 128 | 0.004 |
| L145 *C*. *albicans* | 256 | - | - |

For *Candida albicans,* amphotericin B was used as a control antibiotic and the MIC (µg/ml) was 0.5.

Compound 1 (103/33-F710) showed very potent antibacterial activity against *Staphylococci* including hospital-associated Methicillin-Resistant (HA-MRSA), community-associated Methicillin-Resistant (CA-MRSA), MRSA strains with reduced susceptibility to glycopeptide (GISA) and multi-resistant (MDR) coagulase-negative strains. Compound 1 (103/33-F710) exhibited an high antibacterial activity also against *Listeria monocytogenes, Bacillus cereus* and *Bacillus clausii.* Antibiotic 103/33-F710 was active against recent clinical isolates of *Enterococcus* spp., including strains with vancomycin, ciprofloxacin and linezolid resistance.

The compound was not active against Gram negative bacteria (*E.coli*) and *Candida albicans.*

Cytotoxicity. Compound 1 (103/33-F710) showed no toxicity against human cells at 200 µg/ml, the highest concentration tested (Hela cells from cervix adenocarcinoma, HepG2 cells from hepatocarcinoma and BJ5TA - ATCC CRL-4401 normal fibroblast). The compound showed no hemolytic activity against human erythrocytes.

Effectiveness. The activity of Compound 1 (103/33-F710) was demonstrated in a murine septicemia model using *Staphylococcus aureus* Smith 819 ATCC 19636. 103/33-F710 was effective for treating the infection with an ED50 of 15 mg/kg. This level of protection was observed after intravenous administrations.

### EXAMPLE 8: Purification protocol for compound 2 (103/33-F696)

Mycelium/supernatant separation was obtained by centrifugation. The supernatant (10.5 L) was acidified under stirring with an aqueous solution of 1N hydrochloric acid (140 ml) to pH 3.0. Precipitation of a pellet occurred; after centrifugation the supernatant was discarded and the pellet was collected. This was then washed to remove salts/impurities, first with H₂O and then with acetonitrile. This pellet was then extracted several times with acetonitrile added with 5% formic acid (5 × 1.5 L), which was successively dried under evaporation. The crude obtained solid (1.6 g) contained compound 2 (103/33 F-696) in a 4-5% purity range. The solution was filtered and evaporated to dryness.

Compound 2 (103/33-F696) was then separated from other factors by purification on a Phenomenex Luna Preparative C18 column 5µ (250 × 21.2 mm) (Phenomenex, Torrance CA) using two subsequent column runs. Starting crude obtained above (1.6 g) was dissolved in DMSO:H₂O:CH₃CN 1:1:1 (v/v, 60 ml) then 4 ml were injected using a 51 minutes stepwise gradient elution following method 2 from Example 1. Phase B was CH₃CN +0.1% TFA. Phase A was H₂O +0.1% TFA. The process was repeated for 15 times. Mixed fractions of compound 1 ( 103/33 F-710) and compound 2 (103/33-F696) were pooled and evaporated to dryness. The obtained crude enriched with compound 2 (103/33-F696) was redissolved and injected in the same column system following the same method. Pure fractions of factor compound 2 (103/33-F696) were pooled and acetonitrile was evaporated, water was extracted with n-BuOH, which was separated and concentrated to minimal volume. Diethyl ether was then added until precipitation of a red solid occurs. This was filtered, washed and dried under vacuum. Yield: 33 mg. Compound 2 (103/33-F696): red powder; UV (MeOH) λ max 287, 333 and 473 nm; MS (ESI) m/z 696.17 (M + H+); 1H-NMR (500 MHz, DMSO): δ (ppm) 14.42 (bs, 1H), 11.93 (bs, 1H), 11.49 (bs, 1H), 9.11 (s, 1H), 8.25 (s, 1H), 8.06 (s, 2H), 7.08 (s, 1H), 6.75 (s, 1H), 6.50 (s, 1H), 5.06 (m, 1H), 4.14 (m, 1H), 4.00 (s, 3H), 3.95 (s, 3H), 3.87 (m, 1H), 3.40 (m, 1H), 2.91 (s, 3H), 2.77 (m, 1H), 2.61 (s, 3H), 2.09 (m, 1H); 13C-NMR (500 MHz, DMSO): δ (ppm) 189.38, 182.56, 182.14, 180.91, 170.13, 166.29, 165.01, 162.8, 158.56, 158.28, 157.11, 155.75, 151.10, 143.86, 136.00, 131.19, 130.56, 130.39, 129.34, 126.5, 120.97, 110.37, 119.21, 110.06, 109.14, 108.49, 101.96, 62.60, 62.25, 56.94, 55.64, 51.24, 35.53, 31.76, 22.59.

### Example 9

### Biological activity of compound 2 (103/33-F696)

Antibacterial activity. The antimicrobial activity of the antibiotic 103/33-F696 was determined by the broth microdilution method according to the procedures disclosed in Example 7. The results are shown in table 6.

**Table 6: Antimicrobial activity reported as MIC (µg/ml) of compound 2 (103/33-F696)**

| MIC (µg/ml) | | | |
|---|---|---|---|
| | **103/33** - **696** | **Vancomycin** | **Ciprofloxacin** |
| L1400 *S.aureus* HA- MRSA | 0.03 | 1 | 0.125 |
| L100 *S.aureus* ATCC6538P | 0.03 | 1 | 0.25 |
| L819 *S. aureus* Smith | 0.015 | 1 | 0.06 |
| L1577 *S. epidermidis* | 0.03 | 1 | 0.06 |
| L1730 *S. haemolyticus* | 0.03 | 0.5 | 0.06 |
| L1729 *S. haemolyticus* MDR | 0.06 | 2 | - |
| L49 *S*. *pyogenes* C203 | 1 | 0.5 | 0.25 |
| L44 *S*. *pneumoniae* | 32 | 0.5 | 0.25 |
| L568 *E. faecium* | 0.125 | 2 | 2 |
| L569 *E. faecium* VanA | 0.5 | > 128 | 2 |
| L559 *E. faecalis* | 0.03 | 0.5 | 0.25 |
| L560 *E. faecalis* VanA | 0.03 | > 128 | 0.25 |
| L3294 M. *catarrhalis* | 64 | 16 | 0.06 |
| L47 *E. coli* | >512 | > 128 | 0.004 |
| L145 *C*. *albicans* | >512 | - | - |

For *Candida albicans,* amphotericin B was used as a control antibiotic and the MIC (µg/ml) was 0.5.

Compound 2 (103/33-F696) showed very potent antibacterial activity against *Staphylococci* including hospital-associated Methicillin-Resistant (HA-MRSA). Antibiotic 103/33-F696 was active against recent clinical isolates of *Enterococcus* spp., including strains with vancomycin resistance.

The compound was not active against Gram negative bacteria (*E.coli*) and *Candida albicans.*

### EXAMPLE 10: Purification protocol for compound 3 (103/33-F712)

Mycelium/supernatant separation was obtained by centrifugation. The supernatant (10.5 L) was acidified under stirring with an aqueous solution of 1N hydrochloric acid (140 ml) to pH 3.0. Precipitation of a pellet occurred; after centrifugation the supernatant was discarded and the pellet was collected. This was then washed to remove salts/impurities, first with H₂O and then with acetonitrile. This pellet was then extracted several times with acetonitrile added with 5% formic acid (5 × 1.5 L), which was successively dried under evaporation. The crude obtained solid (1.6 g) contained 103/33 F-712 in a 8-10% purity range.

Compound 3 (103/33-F712) was then separated from other factors by purification on a Phenomenex Luna Preparative C18 column 5µ (250 × 21.2 mm) (Phenomenex, Torrance CA) using a gradient method. Starting crude obtained above (1.6 g) was dissolved in DMSO:H₂O:CH₃CN 1:1:1 (v/v, 60 ml) then 4 ml were injected using a 51 minutes stepwise gradient elution method 3 from Example 1. Phase B was CH₃CN +0.1% TFA. Phase A was H₂O +0.1% TFA. Process was repeated for 15 times. Pure fractions containing compound 3 (103/33-F712) were pooled and acetonitrile was evaporated, water was extracted with n-BuOH, which was separated and concentrated to minimal volume. Diethyl ether was then added until precipitation of a dark brown solid occurs. This was filtered, washed and dried under vacuum. Yield: 40 mg.

Compound 3 (103/33-F712): brown powder, UV (MeOH) λ max 297, 426 nm; MS (ESI) m/z 713.20 (M + H+); 1H-NMR (500 MHz, DMSO): δ (ppm) 14.89 (s, 1H), 13.34 (s, 1H), 11.15 (s, 1H), 10.36 (s, 1H), 9.85 (s, 1H), 8.10 (s, 1H), 8.00 (s, 2H), 7.12 (s, 1H), 7.03 (s, 1H), 6.60 (s, 1H), 5.12 (m, 1H), 4.16 (m, 1H), 4.12 (s, 3H), 3.99 (s, 3H), 3.96 (s, 3H), 3.85 (m, 1H), 3.41 (m, 1H), 2.89 (s, 3H), 2.79 (m, 1H), 2.55 (s, 3H), 2.12 (m, 1H).

### Example 11

### Biological activity of compound 3 (103/33-F712)

Antibacterial activity. The antimicrobial activity of the antibiotic 103/33-F712 was determined by the broth microdilution method according to the procedures disclosed in Example 7. The results are shown in table 7.

**Table 7: Antimicrobial activity reported as MIC (µg/ml) of ID103/33-F712**

| MIC (µg/ml) | | | |
|---|---|---|---|
| | **103/33 - 712** | **Vancomycin** | **Ciprofloxacin** |
| L1400 *S.aureus* HA- MRSA | 0.25 | 1 | 0.125 |
| L100 *S.aureus* ATCC6538P | 0.25 | 1 | 0.25 |
| L819 *S. aureus* Smith | 0.25 | 1 | 0.06 |
| L1577 *S. epidermidis* | 0.25 | 1 | 0.06 |
| L1730 *S*. *haemolyticus* | 0.5 | 0.5 | 0.06 |
| L1729 *S. haemolyticus* MDR | 0.5 | 2 | - |
| L49 *S*. *pyogenes* C203 | 32 | 0.5 | 0.25 |
| L44 *S*. *pneumoniae* | 64 | 0.5 | 0.25 |
| L568 *E. faecium* | 4 | 2 | 2 |
| L569 *E. faecium* VanA | 8 | > 128 | 2 |
| L569 *E. faecium* VanA 30 % SB | - | > 128 | 2 |
| L559 *E. faecalis* | 0.25 | 0.5 | 0.25 |
| L560 *E. faecalis* VanA | 0.25 | > 128 | 0.25 |
| L3294 *M*. *catarrhalis* | 128 | 16 | 0.06 |
| L47 *E. coli* | >512 | > 128 | 0.004 |
| L145 *C*. *albicans* | >512 | - | - |

For *Candida albicans,* amphotericin B was used as a control antibiotic and the MIC (µg/ml) was 0.5.

Compound 3 (103/33-F712) showed antibacterial activity against *Staphylococci* including hospital-associated Methicillin-Resistant (HA-MRSA). Antibiotic 103/33-F712 was active against recent clinical isolates of *Enterococcus* spp., including strains with vancomycin resistance.

The compound was not active against Gram negative bacteria (*E.coli*) and *Candida albicans.*

### Example 12: Preparation of compound 4 (Factor 103/33-F710a)

Compound 1 obtained by example 3 has been treated in conditions known by the skilled man in the art to transform the exocyclic C-N bonds of compound 1 into endocyclic C-N bonds in order to obtain compound 4. Compound 4 is the isomeric form of compound 1.

Compound 4: MS (ESI) m/z 711.19 (M + H+).

### Example 13: Preparation of compound 5 (Factor 103/33-F696a)

Compound 2 obtained by example 8 has been treated in conditions known by the skilled man in the art to transform the exocyclic C-N bonds of compound 2 into endocyclic C-N bonds in order to obtain compound 5. Compound 5 is the isomeric form of compound 2.

Compound 5: MS (ESI) m/z 696.17 (M + H+).

### Example 14: Preparation of compound 6 (Factor 103/33-F712a)

Compound 3 obtained by example 10 has been treated in conditions known by the skilled man in the art to transform the exocyclic C-N bonds of compound 3 into endocyclic C-N bonds in order to obtain compound 6. Compound 6 is the isomeric form of compound 3.

Compound 3: MS (ESI) m/z 713.20 (M + H+).

## Claims

1. A compound of Formula (I) 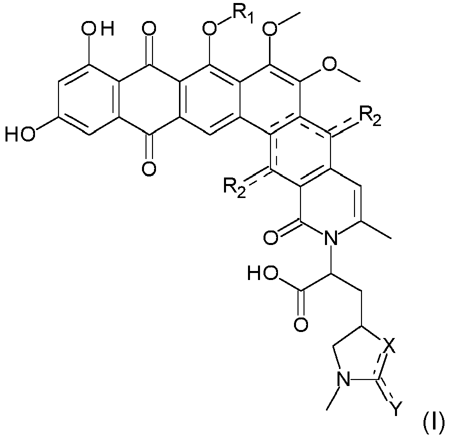 or a pharmaceutically acceptable salt thereof
wherein
R₁ is H or CH₃,
R₂ is O or OH,
the dotted line represents the presence of a single or a double bond, and
X is N or NH and Y is NH or NH₂.

2. The compound of Formula (I) according to claim 1, selected from the group consisting of:
2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionic acid (compound 1 of Formula (I): factor 103/33-F710) 2-(8,10,12-Trihydroxy-6,7,dimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionic acid (compound 2 of Formula (I): factor 103/33-F696) 2-(5,10,12,16-tetrahydroxy-6,7,8-trimethoxy-3-methyl-1,9,14,trioxo-,9,14, -dihydro-1H-2-aza-hexaphen-2-yl)-3-(2-imino-1-methyl-imidazolidin-4-yl)-propionic acid (compound 3 of Formula (I): Factor103/33-F712);
2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-amino-1-methyl-imidazol-4-yl)-propionic acid (compound 4 of Formula (I): Factor103/33-F710a);
2-(8,10,12-TriDihydroxy-6,7-dimethoxy-3-methyl-1,5,9,14,16-pentaoxo-5,9,14,16-tetrahydro-1H-2-aza-hexaphen-2-yl)-3-(2-amino-1-methyl-imidazol-4-yl)-propionic acid (compound 5 of Formula (I) Factor103/33-F696a); and
2-(5,10,12,16-tetrahydroxy-6,7,8-trimethoxy-3-methyl-1,9,14-trioxo-9,14,-dihydro-1H-2-aza-hexaphen-2-yl)-3-(2-amino-1-methyl-imidazol-4-yl)-propionic acid (compound 6 of Formula (I) Factor103/33-F712a).

3. A pharmaceutical composition comprising a compound of Formula (I) according to any one of claims 1 to 2, and a pharmaceutically acceptable excipient.

4. A process for producing a compound of Formula (I) according to any one of claims 1 to 2, by using a microorganism *Nonomuraea* FIIRV sp. 103/33 deposited on January 19, 2015 at the Deutsche Sammlung von Mikroorganismen und Zellkulturen GmbH, (DSMZ) Inhoffenstraße 7B, 38124 Braunschweig, Deutschland under Budapest Treaty under accession number 29890.

5. The process according to claim 4, comprising the steps of:
(a) cultivating the strain *Nonomuraea* FIIRV sp. 103/33 DSM 29890, under aerobic conditions, in an aqueous nutrient medium comprising a source of carbon, nitrogen and inorganic salts, thus obtaining a culture fermentation broth comprising one or more compounds of Formula (I);
(b) isolating the compound of Formula (I) from the culture fermentation broth comprising one or more compounds of Formula (I); and
(c) purifying the isolated compound of Formula (I).

6. The process according to claim 5, comprising the step of pre-cultering the strain *Nonomuraea* FIIRV sp. 103/33 DSM 29890 before step a).

7. The process according to any one of claims 5 and 6, wherein the isolating step b) is carried out by centrifuging or filtering the fermentation broth and recovering the antibiotic from the supernatant or filtered broth according to at least one technique selected from: extraction with a water-immiscible solvent, precipitation by adding a non-solvent or by changing the pH of the solution, absorption chromatography, partition chromatography, reverse phase partition chromatography, ion exchange chromatography, molecular exclusion chromatography, or a combination of two or more of said techniques.

8. The process according to any one of claims 5 and 6, wherein the isolating step b) is carried out by separating the mycelium from the supernatant of the fermentation broth and extraction of the mycelium with a water-miscible solvent whereby, after the removal of the spent mycelium, a water-miscible solution containing the crude antibiotic is obtained, which is processed to recover the compound of Formula (I) by means of a technique selected from at least one of: extraction with a solvent, precipitation by adding a non-solvent or by changing the pH of the solution, absorption chromatography, partition chromatography, reverse phase partition chromatography, ion exchange chromatography and molecular exclusion chromatography, or a combination of two or more of said techniques.

9. The process according to any one of claims 5 and 6, wherein the isolating step b) is carried out on the whole strain culture by adding an absorbing resin, maintaining the obtained mixture under stirring until the antibiotic product is almost entirely bound to the resin, separating the resin and mycelium by filtration or centrifugation and submitting said separated material to extraction with a water-miscible solvent.

10. A microorganism *Nonomuraea* FIIRV sp. 103/33 DSM 29890 as number DSM 29890 on January 19, 2015 in the Deutsche Sammlung von Mikroorganismen und Zellkulturen GmbH, (DSMZ) Inhoffenstraße 7B, 38124 Braunschweig, Deutschland under Budapest Treaty.

11. A compound of Formula (I) according to any one of claims 1 to 2, or its pharmaceutically acceptable salt for use as a medicament.

12. The compound for use according to claim 11, wherein the compound is orally administered in the form of tablets, capsules, lozenges, liquid solutions or suspensions.

13. A compound of Formula (I) according to any one of claims 1 to 2, or its pharmaceutically acceptable salt for use in treating bacterial infectious diseases.

14. The compound for use according to claim 13, wherein said bacterial infectious diseases are caused by Gram-positive bacteria, preferably infections caused by Gram-positive susceptible, resistant and multi-resistant pathogenic bacteria.

15. The compound of Formula (I) for use according to claim 14, wherein said bacterial infectious diseases are caused by bacteria of at least one of the following genera: *Enterococci, Staphylococci, Listeriae* and *Bacilli.*

## Patentansprüche

1. Verbindung der Formel (I) 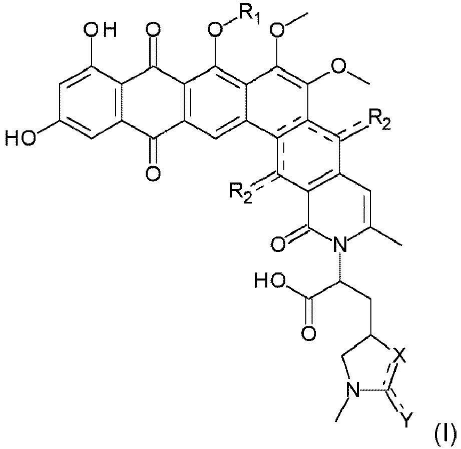 oder pharmazeutisch akzeptables Salz derselben,
wobei
R₁ H oder CH₃ ist,
R₂ O oder OH ist,
die gestrichelte Linie das Vorhandensein einer Einfach- oder einer Doppelbindung darstellt, und
X N oder NH ist, und Y NH oder NH₂ ist.

2. Verbindung der Formel (I) nach Anspruch 1, ausgewählt aus der Gruppe bestehend aus:
2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionsäure (Verbindung 1 der Formel (I): Faktor 103/33-F710);
2-(8,10,12-Trihydroxy-6,7-dimethoxy-3-methyl-1,5,9,14,16-pentaoxo-5,9,14,16-tetrahydro-1H-2-aza-hexaphen-2-yl)-3-(2-imino-1-methyl-imidazolidin-4-yl)-propionsäure (Verbindung 2 der Formel (I): Faktor 103/33-F696);
2-(5,10,12,16-Tetrahydroxy-6,7,8-trimethoxy-3-methyl-1,9,14-trioxo-9,14-dihydro-1H-2-aza-hexaphen-2-yl)-3-(2-imino-1-methyl-imidazolidin-4-yl)-propionsäure (Verbindung 3 der Formel (I): Faktor 103/33-F712);
2-(10,12-Dihydroxy-6,7,8-trimethoxy-3-methyl-1,5,9,14,16-pentaoxo-5,9,14,16-tetrahydro-1H-2-aza-hexaphen-2-yl)-3-(2-amino-1-methylimidazol-4-yl)-propionsäure (Verbindung 4 der Formel (I): Faktor 103/33-F710a);
2-(8,10,12-TriDihydroxy-6,7-dimethoxy-3-methyl-1,5,9,14,16-pentaoxo-5,9,14,16-tetrahydro-1H-2-aza-hexaphen-2-yl)-3-(2-amino-1-methylimidazol-4-yl)-propionsäure (Verbindung 5 der Formel (I): Faktor 103/33-F696a); und
2-(5,10,12,16-Tetrahydroxy-6,7,8-trimethoxy-3-methyl-1,9,14-trioxo-9,14-dihydro-1H-2-aza-hexaphen-2-yl)-3-(2-amino-1-methyl-imidazol-4-yl)-propionsäure (Verbindung 6 der Formel (I): Faktor 103/33-F712a).

3. Pharmazeutische Zusammensetzung, umfassend eine Verbindung der Formel (I) nach einem der Ansprüche 1 bis 2 und einen pharmazeutisch akzeptablen Hilfsstoff.

4. Verfahren zur Herstellung einer Verbindung der Formel (I) nach einem der Ansprüche 1 bis 2 unter Verwendung eines Mikroorganismus *Nonomuraea* FIIRV sp. 103/33, hinterlegt am 19. Januar 2015 bei der Deutschen Sammlung von Mikroorganismen und Zellkulturen GmbH (DSMZ), Inhoffenstraße 7B, 38124 Braunschweig, Deutschland, gemäß dem Budapester Vertrag, unter der Eingangsnumer 29890.

5. Verfahren nach Anspruch 4, umfassend die Schritte:
(a) Kultivieren des Stamms *Nonomuraea* FIIRV sp. 103/33 DSM 29890 unter aeroben Bedingungen in einem wässrigen Nährmedium, das eine Quelle von Kohlenstoff, Stickstoff und anorganischer Salze umfasst, dadurch Erhalten einer Kulturfermentationsbrühe, die eine oder mehrere Verbindungen der Formel (I) umfasst;
(b) Isolieren der Verbindung der Formel (I) aus der Kulturfermentationsbrühe, die eine oder mehrere Verbindungen der Formel (I) umfasst; und
(c) Reinigen der isolierten Verbindung der Formel (I).

6. Verfahren nach Anspruch 5, umfassend den Schritt des Vor-Kultivierens des Stamms *Nonomuraea* FIIRV sp. 103/33 DSM 29890 vor Schritt a).

7. Verfahren nach einem der Ansprüche 5 und 6, wobei der Isolierungsschritt b) durchgeführt wird, indem die Fermentationsbrühe zentrifugiert oder filtriert wird und das Antibiotikum aus dem Überstand oder der filtrierten Brühe nach mindestens einer Methode gewonnen wird, welche ausgewählt ist aus: Extraktion mit einem nicht mit Wasser mischbaren Lösungsmittel, Ausfällung durch Zugabe eines Nichtlösungsmittels oder durch Ändern des pH der Lösung, Absorptionschromatographie, Verteilungschromatographie, Umkehrphasen-Verteilungschromatographie, lonenaustauschchromatographie, Größenausschlusschromatographie oder einer Kombination zweier oder mehrerer der genannten Methoden.

8. Verfahren nach einem der Ansprüche 5 und 6, wobei der Isolierungsschritt b) durchgeführt wird durch Abtrennen des Mycels von dem Überstand der Fermentationsbrühe und Extraktion des Mycels mit einem mit Wasser mischbaren Lösungsmittel, wodurch nach Entfernung des verbrauchten Mycels eine mit Wasser mischbare, das rohe Antibiotikum enthaltende Lösung erhalten wird, die verarbeitet wird, um die Verbindung der Formel (I) mittels einer Methode zu gewinnen, die ausgewählt ist aus mindestens einer von: Extraktion mit einem Lösungsmittel, Ausfällung durch Zugabe eines Nichtlösungsmittels oder durch Ändern des pH der Lösung, Absorptionschromatographie, Verteilungschromatographie, Umkehrphasen-Verteilungschromatographie, lonenaustauschchromatographie und Größenausschlusschromatographie, oder einer Kombination zweier oder mehrerer der genannten Methoden.

9. Verfahren nach einem der Ansprüche 5 und 6, wobei der Isolierungsschritt b) an der gesamten Stammkultur durchgeführt wird, indem ein absorbierendes Harz zugegeben wird, die erhaltene Mischung unter Rühren gehalten wird, bis das antibiotische Produkt fast vollständig an das Harz gebunden ist, das Harz und das Mycel durch Filtration oder Zentrifugieren getrennt werden, und das abgetrennte Material einer Extraktion mit einem mit Wasser mischbaren Lösungsmittel unterzogen wird.

10. Mikroorganismus *Nonomuraea* FIIRV sp. 103/33 als Nummer DSM 29890 am 19. Januar 2015 in der Deutschen Sammlung von Mikroorganismen und Zellkulturen GmbH (DSMZ), Inhoffenstraße 7B, 38124 Braunschweig, Deutschland, gemäß dem Budapester Vertrag.

11. Verbindung der Formel (I) nach einem Ansprüche 1 bis 2, oder deren pharmazeutisch akzeptables Salz, zur Verwendung als Medikament.

12. Verbindung zur Verwendung nach Anspruch 11, wobei die Verbindung in Form von Tabletten, Kapseln, Lutschtabletten, flüssigen Lösungen oder Suspensionen oral verabreicht wird.

13. Verbindung der Formel (I) nach einem der Ansprüche 1 bis 2, oder deren pharmazeutisch akzeptables Salz, zur Verwendung in der Behandlung bakterieller Infektionskrankheiten.

14. Verbindung zur Verwendung nach Anspruch 13, wobei die bakteriellen Infektionskrankheiten durch Gram-positive Bakterien verursacht sind, bevorzugt Infektionen, die durch Gram-positive empfindliche, resistente und multiresistente pathogene Bakterien verursacht sind.

15. Verbindung der Formel (I) zur Verwendung nach Anspruch 14, wobei die bakteriellen Infektionskrankheiten durch Bakterien aus mindestens einer der folgenden Gattungen verursacht sind: *Enterococci, Staphylococci, Listeriae* und *Bacilli.*

## Revendications

1. Composé selon la formule (I) 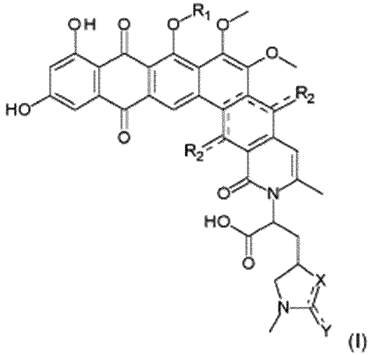 ou un sel pharmaceutique moins acceptable de celui-ci
où
R₁ est H ou CH₃,
R₂ est O ou OH,
la ligne pointillée représente la présence d'une liaison simple ou double, et
X est N ou NH et Y est NH ou NH₂.

2. Composé selon la formule (I) selon la revendication 1, choisi dans le groupe constitué des éléments suivants :
acide 2-(10,12-dihydroxy-6,7,8-triméthoxy-3-méthyl-1,5,9,14,16-pentaoxo-5,9,14,16-tétrahydro-1H-2-aza-hexaphen-2-yl)-3-(2-imino-1-méthyl-imidazolidin-4-yl)propionique (composé 1 selon la formule (I) : facteur 103/33-F710) ;
acide 2-(8,10,12-trihydroxy-6,7,diméthoxy-3-méthyl-1,5,9,14,16-pentaoxo-5,9,14,16-tétrahydro-1H-2-aza-hexaphen-2-yl)-3-(2-imino-1-méthyl-imidazolidin-4-yl)propionique (composé 2 de la formule (I) : facteur 103/33-F696) ;
acide 2-(5,10,12,16-tétrahydroxy-6,7,8-triméthoxy-3-méthyl-1,9,14,trioxo-,9,14,-dihydro-1H-2-aza-hexaphen-2-yl)-3-(2-imino-1-méthyl-imidazolidin-4-yl)propionique (composé 3 selon la formule (I) : facteur 103/33-F712) ;
acide 2-(10,12-dihydroxy-6,7,8-triméthoxy-3-méthyl-1,5,9,14,16-pentaoxo-5,9,14,16-tétrahydro-1H-2-aza-hexaphen-2-yl)-3-(2-amino-1-méthyl-imidazol-4-yl)propionique (composé 4 selon la formule (I) : facteur 103/33-F710a) ;
acide 2-(8,10,12-tridihydroxy-6,7-diméthoxy-3-méthyl-1,5,9,14,16-pentaoxo-5,9,14,16-tétrahydro-1H-2-aza-hexaphen-2-yl)-3-(2-amino-1-méthyl-imidazol-4-yl)propionique (composé 5 selon la formule (I) facteur 103/33-F696a) ; et
acide 2-(5,10,12,16-tétrahydroxy-6,7,8-triméthoxy-3-méthyl-1,9,14-trioxo-9,14,-dihydro-1H-2-aza-hexaphen-2-yl)-3-(2-amino-1-méthyl-imidazol-4-yl)propionique (composé 6 selon la formule (I) facteur 103/33-F712a).

3. Composition pharmaceutique comprenant un composé selon la formule (I) selon l'une quelconque des revendications 1 à 2, et un excipient pharmaceutiquement acceptable.

4. Procédé de production d'un composé selon la formule (I) selon l'une quelconque des revendications 1 à 2, en utilisant un micro-organisme *Nonomuraea* FIIRV sp. 103/33 déposé le 19 janvier 2015 à la Deutsche Sammlung van Mikroorganismen und Zellkulturen GmbH, (DSMZ) Inhoffenstra e 7B, 38124 Brunswick, Allemagne conformément au Traité de Budapest sous le numéro d'ordre 29890.

5. Procédé selon la revendication 4, comprenant en outre les étapes consistant à :
(a) cultiver la souche *Nonomuraea* FIIRV sp. 103/33 DSM 29890, dans des conditions aérobies, dans un milieu nutritif aqueux comprenant une source de carbone, d'azote et de sels inorganiques, obtenant ainsi un bouillon de fermentation de culture comprenant un ou plusieurs composés selon la formule (I) ;
(b) isoler le composé selon la formule (I) à partir du bouillon de culture de fermentation comprenant un ou plusieurs composés selon la formule (I) ; et
(c) purifier le composé isolé selon la formule (I).

6. Procédé selon la revendication 5, comprenant l'étape de pré-culture de la souche *Nonomuraea* FIIRV sp. 103/33 DSM 29890 avant l'étape a).

7. Procédé selon l'une quelconque des revendications 5 et 6, dans lequel l'étape d'isolement b) est mise en œuvre par centrifugation ou filtration du bouillon de fermentation et récupération de l'antibiotique à partir du surnageant ou du bouillon filtré selon au moins une technique choisie parmi : l'extraction avec un solvant non miscible à l'eau, la précipitation par ajout d'un non-solvant ou par modification du pH de la solution, la chromatographie d'absorption, la chromatographie de partage, la chromatographie de partage en phase inverse, la chromatographie d'échange d'ions, la chromatographie d'exclusion moléculaire, ou une combinaison de deux de ces techniques ou plus.

8. Procédé selon l'une quelconque des revendications 5 et 6, dans lequel l'étape d'isolement b) est mise en œuvre en séparant le mycélium du surnageant du bouillon de fermentation et en extrayant le mycélium avec un solvant miscible à l'eau, moyennant quoi, après l'élimination du mycélium usé, une solution miscible à l'eau contenant l'antibiotique brut est obtenue, qui est traitée pour récupérer le composé selon la formule (I) au moyen d'une technique choisie parmi au moins l'une des techniques suivantes : l'extraction avec un solvant, la précipitation par ajout d'un non-solvant ou par modification du pH de la solution, la chromatographie d'absorption, la chromatographie de partage, la chromatographie de partage en phase inverse, la chromatographie d'échange d'ions et la chromatographie d'exclusion moléculaire, ou une combinaison de deux de ces techniques ou plus.

9. Procédé selon l'une quelconque des revendications 5 et 6, dans lequel l'étape d'isolement b) est mise en œuvre sur la culture entière de la souche en ajoutant une résine absorbante, en maintenant le mélange obtenu sous agitation jusqu'à ce que le produit antibiotique soit presque entièrement lié à la résine, en séparant la résine et le mycélium par filtration ou centrifugation et en soumettant ledit matériau séparé à une extraction avec un solvant miscible à l'eau.

10. Micro-organisme *Nonomuraea* FIIRV sp. 103/33 DSM 29890 sous le numéro DSM 29890 le 19 janvier 2015 dans la Deutsche Sammlung van Mikroorganismen und Zellkulturen GmbH, (DSMZ) Inhoffenstra e 7B, 38124 Brunswick, Allemagne conformément au Traité de Budapest.

11. Composé selon la formule (I) selon l'une quelconque des revendications 1 à 2, ou son sel pharmaceutiquement acceptable pour une utilisation en tant que médicament.

12. Composé pour une utilisation selon la revendication 11, dans lequel le composé est administré par voie orale sous la forme de comprimés, de gélules, de pastilles, de solutions liquides ou de suspensions.

13. Composé selon la formule (I) selon l'une quelconque des revendications 1 à 2, ou son sel pharmaceutiquement acceptable pour une utilisation dans le traitement de maladies infectieuses bactériennes.

14. Composé pour une utilisation selon la revendication 13, dans lequel lesdites maladies infectieuses bactériennes sont causées par des bactéries Gram-positives, de préférence des infections causées par des bactéries pathogènes Gram-positives sensibles, résistantes et multi-résistantes.

15. Composé selon la formule (I) pour une utilisation selon la revendication 14, dans lequel lesdites maladies infectieuses bactériennes sont causées par des bactéries d'au moins l'un des genres suivants : *Enterococci, Staphylococci, Listeriae* et *Bacilli.*
